# EUROPEAN PATENT SPECIFICATION

(11) **EP 3 843 748 B1**
(45) Date of publication and mention of the grant of the patent: **25.06.2025**
(21) Application number: 19762358.0
(22) Date of filing: 30.08.2019
(51) Int. Cl.: A61K 31/635, A61K 45/06, A61K 47/68, A61P 35/00

(54) **COMBINATION THERAPY**
KOMBINATIONSTHERAPIE
POLYTHÉRAPIE

(30) Priority: 31.08.2018 GB 201814207; 10.06.2019 GB 201908225
(43) Date of publication of application: 07.07.2021
(73) Proprietor: ADC Therapeutics SA, 1066 Epalinges (CH); MedImmune Limited, Cambridge Cambridgeshire CB21 6GH (GB)
(72) Inventor: ZAMMARCHI, Francesca, 1066 Epalinges (CH); BERTONI, Francesco, 6500 Bellizona (CH)
(74) Representative: Mewburn Ellis LLP
(86) International application number: PCT/EP2019/073212
(87) International publication number: WO 2020/043878

(56) References cited:
- WO-A1-2014/057117
- WO-A1-2017/160954
- WO-A1-2018/078123
- WO-A1-2018/193105
- WO-A1-2018/229222
- C TARANTELLI ET AL: "The antibody-drug conjugate (ADC) loncastuximab tesirine (ADCT-402) targeting CD19 shows strong in vitro anti-lymphoma activity both as single agents and in combination - 15th International Conference on Malignant Lymphoma", HEMATOLOGICAL ONCOLOGY, vol. 37, no. Suppl.2, 1 June 2019 (2019-06-01), pages 129 - 130, XP055646201, DOI: 10.1002/hon.90_2629

## Description

### EARLIER APPLICATIONS

The present application claims priority from United Kingdom application GB1814207.5 filed on 31 August 2018, and United Kingdom application GB1908225.4 filed on 10 June 2019.

### FIELD

The present disclosure relates to combination therapies for the treatment of pathological conditions, such as cancer. In particular, the present disclosure relates to combination therapies comprising treatment with an anti-CD19 Antibody Drug Conjugate (anti-CD19 ADC) and an anti-BCL-2 agent as defined in the claims.

### BACKGROUND

### Antibody Therapy

Antibody therapy has been established for the targeted treatment of subjects with cancer, immunological and angiogenic disorders (Carter, P. (2006) Nature Reviews Immunology 6:343-357). The use of antibody-drug conjugates (ADC), i.e. immunoconjugates, for the local delivery of cytotoxic or cytostatic agents, i.e. drugs to kill or inhibit tumour cells in the treatment of cancer, targets delivery of the drug moiety to tumours, and intracellular accumulation therein, whereas systemic administration of these unconjugated drug agents may result in unacceptable levels of toxicity to normal cells (Xie et al (2006) Expert. Opin. Biol. Ther. 6(3):281-291; Kovtun et al (2006) Cancer Res. 66(6):3214-3121; Law et al (2006) Cancer Res. 66(4):2328-2337; Wu et al (2005) Nature Biotech. 23(9):1137-1145; Lambert J. (2005) Current Opin. in Pharmacol. 5:543-549; Hamann P. (2005) Expert Opin. Ther. Patents 15(9):1087-1103; Payne, G. (2003) Cancer Cell 3:207-212; Trail et al (2003) Cancer Immunol. Immunother. 52:328-337; Syrigos and Epenetos (1999) Anticancer Research 19:605-614).

### CD19

CD19 is a 95 kDa membrane receptor that is expressed early in B cell differentiation and continues to be expressed until the B cells are triggered to terminally differentiate (Pezzutto et al.(1987), J. Immunol 138:2793; Tedder et al (1994) Immunol Today 15:437). The CD19 extracellular domain contains two C2-type immunoglobulin (IG)-like domains separated by a smaller potentially disulfide-linked domain. The CD19 cytoplasmic domain is structurally unique, but highly conserved between human, mouse, and guinea pig (Fujimoto et al., (1998) Semin Immunol.10:267). CD19 is part of a protein complex found on the cell surface of B-lymphocytes. The protein complex includes CD19, CD21 (complement receptor, type 2), CD81 (TAPA-1), and CD225 (Leu-13) (Fujimoto, supra).

CD19 is an important regulator of transmembrane signals in B cells. An increase or decrease in the cell surface density of CD19 affects B cell development and function, resulting in diseases such as autoimmunity or hypogammaglobulinemia. The CD19 complex potentiates the response of B cells to antigen in vivo through cross-linking of two separate signal transduction complexes found on B cell membranes. The two signal transduction complexes, associated with membrane IgM and CD19, activate phospholipase C (PLC) by different mechanisms. CD19 and B cell receptor cross-linking reduces the number of IgM molecules required to activate PLC. CD19 also functions as a specialized adapter protein for the amplification of Arc family kinases (Hasegawa et ah, (2001) J Immunol 167:3190).

CD19 binding has been shown to both enhance and inhibit B-cell activation and proliferation, depending on the amount of cross-linking that occurs (Tedder, 1994, Immunol. Today 15:437). CD19 is expressed on greater than 90% of B-cell lymphomas and has been predicted to affect growth of lymphomas *in vitro* and *in vivo.*

### Therapeutic uses of anti-CD19 ADCs

The efficacy of an Antibody Drug Conjugate comprising an anti-CD19 antibody (an anti-CD19-ADC) in the treatment of, for example, cancer has been established - see, for example, WO2014/057117 and WO2016/166298.

Research continues to further improve the efficacy, tolerability, and clinical utility of anti-CD19 ADCs. To this end, the present authors have identified clinically advantageous combination therapies in which an anti-CD19 ADC is administered in combination with at least one anti-BCL-2 agent.

### SUMMARY

The present invention relates to the combined use of an anti-CD19 ADC which is ADCx19 and an anti-BCL-2 agent which is venetoclax for use in the treatment of cancer.

The references to methods of treatment in the summary and in the detailed description of the invention in this description are to be interpreted as references to the compounds, pharmaceutical compositions and medicaments of the present invention for use in a method for treatment of the human (or animal) body by therapy.

The present authors have determined that the administration of a combination of an anti-CD19 ADC and anti-BCL-2 agent to an individual leads to unexpected clinical advantages. The present authors have further determined that administration of an anti-CD19 ADC to an individual that has either been treated with, or is being treated with, and anti-BCL-2 agent leads to a synergistic increase in treatment efficacy.

Accordingly, in a first aspect the present disclosure provides a method of treating cancer, wherein an individual is selected for treatment with the anti-CD19 ADC if the individual has been treated, or is being treated, with the anti-BCL-2 agent, which is venetoclax. The individual may be selected for treatment if the individual is refractory to treatment, or further treatment, with the anti-BCL-2 agent.

In another aspect, the present disclosure provides a method for treating cancer in an individual, the method comprising selecting an individual as suitable for treatment by a method of the first aspect, and then administering to the individual an effective amount of the anti-CD19 ADC and venetoclax.

In another aspect the disclosure provides a method for treating cancer in an individual, the method comprising administering to the individual an effective amount of the anti-CD19 ADC and the anti-BCL-2 agent. The individual may be selected for treatment according to a method according of the first aspect.

The cancer may be non-Hodgkin's Lymphoma, including diffuse large B-cell lymphoma (DLBCL), follicular lymphoma, (FL), Mantle Cell lymphoma (MCL), chronic lymphatic lymphoma (CLL), and Marginal Zone B-cell lymphoma (MZBL), and leukemias such as Hairy cell leukemia (HCL), Hairy cell leukemia variant (HCL-v), and Acute Lymphoblastic Leukaemia (ALL) such as Philadelphia chromosome-positive ALL (Ph+ALL) or Philadelphia chromosome-negative ALL (Ph-ALL).

The anti-CD19-ADC is ADCX19 described herein.

In a non-claimed aspect, the anti-BCL-2 agent may be navitoclax (ABT-263), ABT-737, S55746/BCL201, and oblimersen (G3139). In the present invention, the anti-BCL-2 agent is Venetoclax (ABT-199).

The individual may be human. The individual may have cancer, or may have been determined to have cancer. The individual may have, or have been determined to have, a CD19+ cancer or CD19+ tumour-associated non-tumour cells, such as CD19+ infiltrating B-cells.

In the disclosed methods the anti-CD19 ADC may be administered before the anti-BCL-2 agent, simultaneous with the anti-BCL-2 agent, or after the anti-BCL-2 agent. The disclosed methods may comprise administering a further chemotherapeutic agent to the individual.

In another aspect, the disclosure provides a first composition comprising the anti-CD19 ADC for use in a method of treating cancer in an individual, wherein the treatment comprises administration of the first composition in combination with a second composition comprising the anti-BCL-2 agent.

Also provided by this aspect is a first composition comprising the anti-BCL-2 agent for use in a method of treating cancer in an individual, wherein the treatment comprises administration of the first composition in combination with a second composition comprising the anti-CD19 ADC.

The cancer may be non-Hodgkin's Lymphoma, including diffuse large B-cell lymphoma (DLBCL), follicular lymphoma, (FL), Mantle Cell lymphoma (MCL), chronic lymphatic lymphoma (CLL), and Marginal Zone B-cell lymphoma (MZBL), and leukemias such as Hairy cell leukemia (HCL), Hairy cell leukemia variant (HCL-v), and Acute Lymphoblastic Leukaemia (ALL) such as Philadelphia chromosome-positive ALL (Ph+ALL) or Philadelphia chromosome-negative ALL (Ph-ALL).

The anti-CD19-ADC is ADCX19 described herein.

In a non-claimed aspect, the anti-BCL-2 agent may be navitoclax (ABT-263), ABT-737, S55746/BCL201, and oblimersen (G3139). In the present invention, the anti-BCL-2 agent is Venetoclax (ABT-199).

The individual may be human. The individual may have cancer, or may have been determined to have cancer. The individual may have, or have been determined to have, a CD19+ cancer or CD19+ tumour-associated non-tumour cells, such as CD19+ infiltrating B-cells.

The first composition may be administered before the second composition, simultaneous with the second composition, or after the second composition. The treatment may comprise administering a further chemotherapeutic agent to the individual.

Another non-claimed aspect of the disclosure provides a kit comprising:
a first medicament comprising an anti-CD19 ADC;
a package insert comprising instructions for administration of the first medicament according to a method of treatment as disclosed herein. The kit may further comprise a second medicament comprising an anti-BCL-2 agent.

Another non-claimed aspect of the disclosure provides a kit comprising:
a first medicament comprising an anti-CD19 ADC;
a second medicament comprising an anti-BCL-2 agent; and, optionally,
a package insert comprising instructions for administration of the first medicament to an individual in combination with the second medicament for the treatment of a disorder.

Also provided by this non-claimed aspect is a kit comprising a medicament comprising an anti-CD19 ADC and a package insert comprising instructions for administration of the medicament to an individual in combination with a composition comprising an anti-BCL-2 agent for the treatment of a disorder.

Further provided by this non-claimed aspect is a kit comprising a medicament comprising an anti-BCL-2 agent and a package insert comprising instructions for administration of the medicament to an individual in combination with a composition comprising an anti-CD19 ADC for the treatment of a disorder.

The disorder may be a proliferative disease, for example a cancer such as non-Hodgkin's Lymphoma, including diffuse large B-cell lymphoma (DLBCL), follicular lymphoma, (FL), Mantle Cell lymphoma (MCL), chronic lymphatic lymphoma (CLL), and Marginal Zone B-cell lymphoma (MZBL), and leukemias such as Hairy cell leukemia (HCL), Hairy cell leukemia variant (HCL-v), and Acute Lymphoblastic Leukaemia (ALL) such as Philadelphia chromosome-positive ALL (Ph+ALL) or Philadelphia chromosome-negative ALL (Ph-ALL).

The anti-CD19 ADC may be ADCX19 as described herein.

The anti-BCL-2 agent may be Venetoclax (ABT-199), navitoclax (ABT-263), ABT-737, S55746/BCL201, and oblimersen (G3139). Preferably the anti-BCL-2 agent is Venetoclax (ABT-199).

The individual may be human. The individual may have cancer, or may have been determined to have cancer. The individual may have, or have been determined to have, a CD19+ cancer or CD19+ tumour-associated non-tumour cells, such as CD19+ infiltrating B-cells.

The medicament or composition comprising the anti-CD19 ADC may be administered before the medicament or composition comprising the anti-BCL-2 agent, simultaneous with the medicament or composition comprising the anti-BCL-2 agent, or after the medicament or composition comprising the anti-BCL-2 agent. The treatment may comprise administering a further chemotherapeutic agent to the individual.

In a yet further non-claimed aspect, the disclosure provides a composition comprising an anti-CD19 ADC and an anti-BCL-2 agent.

Also provided in an aspect of the disclosure is a method of treating cancer in an individual, the method comprising administering to the individual an effective amount of the composition comprising the anti-CD19 ADC and the anti-BCL-2 agent.

Also provided in an aspect of the disclosure is a composition comprising the anti-CD19 ADC and the anti-BCL-2 agent for use in a method of treating cancer in an individual.

Also provided in a non-claimed aspect of the disclosure is a kit comprising composition comprising an anti-CD19 ADC and an anti-BCL-2 agent and a set of instructions for administration of the medicament to an individual for the treatment of a disorder.

The disorder may be a proliferative disease, for example a cancer such as non-Hodgkin's Lymphoma, including diffuse large B-cell lymphoma (DLBCL), follicular lymphoma, (FL), Mantle Cell lymphoma (MCL), chronic lymphatic lymphoma (CLL), and Marginal Zone B-cell lymphoma (MZBL), and leukemias such as Hairy cell leukemia (HCL), Hairy cell leukemia variant (HCL-v), and Acute Lymphoblastic Leukaemia (ALL) such as Philadelphia chromosome-positive ALL (Ph+ALL) or Philadelphia chromosome-negative ALL (Ph-ALL).

The anti-CD19-ADC may be ADCX19 as described herein.

The anti-BCL-2 agent may be Venetoclax (ABT-199), navitoclax (ABT-263), ABT-737, S55746/BCL201, and oblimersen (G3139). Preferably the anti-BCL-2 agent is Venetoclax (ABT-199).

The individual may be human. The individual may have cancer, or may have been determined to have cancer. The individual may have, or have been determined to have, a CD19+ cancer or CD19+ tumour-associated non-tumour cells, such as CD19+ infiltrating B-cells.

The treatment may comprise administering a further chemotherapeutic agent to the individual.

### DETAILED DESCRIPTION

### Antibody Drug Conjugates (ADCs)

The present disclosure relates to the improved efficacy of combinations of an ADC and an anti-BCL-2 agent.

The ADC can deliver a drug to a target location. The target location is preferably a proliferative cell population. The antibody is an antibody for an antigen present on a proliferative cell population. In one aspect the antigen is absent or present at a reduced level in a non-proliferative cell population compared to the amount of antigen present in the proliferative cell population, for example a tumour cell population.

The ADC may comprise a linker which may be cleaved so as to release the drug at the target location. The drug may be a compound selected from RelA, RelB, ReIC, RelD or RelE. Thus, the conjugate may be used to selectively provide a compound RelA, RelB, Rel C, RelD or RelE to the target location.

The linker may be cleaved by an enzyme present at the target location.

The disclosure particularly relates treatment with an anti-CD19 ADC disclosed in WO2014/057117, and as herein described.

### anti-CD19 ADCs

As used herein, the terms "anti-CD19 ADC" or "CD19-ADC" refers to an ADC in which the antibody component is an anti-CD19 antibody. The term "PBD-ADC" refers to an ADC in which the drug component is a pyrrolobenzodiazepine (PBD) warhead. The term "anti-CD19-ADC" refers to an ADC in which the antibody component is an anti-CD19 antibody, and the drug component is a PBD warhead. The term "the anti-CD19-ADC" refers to the anti-CD19 ADC of the present invention, i.e. ADCx19.

In non-claimed aspects, the ADC may comprise a conjugate of formula L - (D^{L})ₚ, where D^{L} is of formula I or **II:** 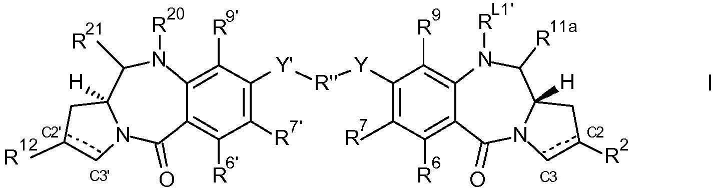 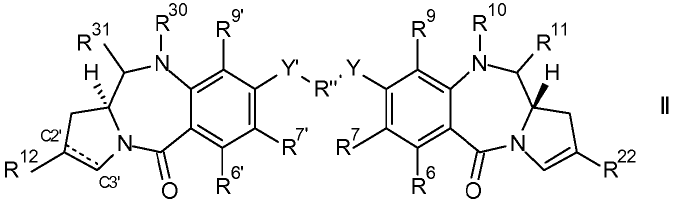 wherein:
L is an antibody (Ab) which is an antibody that binds to CD19;
when there is a double bond present between C2' and C3', R¹² is selected from the group consisting of:
(ia) C₅₋₁₀ aryl group, optionally substituted by one or more substituents selected from the group comprising: halo, nitro, cyano, ether, carboxy, ester, C₁₋₇ alkyl, C₃₋₇ heterocyclyl and bis-oxy-C₁₋₃ alkylene;
(ib) C₁₋₅ saturated aliphatic alkyl;
(ic) C₃₋₆ saturated cycloalkyl;
(id) 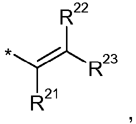 wherein each of R²¹, R²² and R²³ are independently selected from H, C₁₋₃ saturated alkyl, C₂₋₃ alkenyl, C₂₋₃ alkynyl and cyclopropyl, where the total number of carbon atoms in the R¹² group is no more than 5;
(ie) 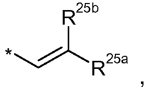 wherein one of R^{25a} and R^{25b} is H and the other is selected from: phenyl, which phenyl is optionally substituted by a group selected from halo, methyl, methoxy; pyridyl; and thiophenyl; and
(if) 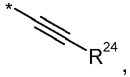 where R²⁴ is selected from: H; C₁₋₃ saturated alkyl; C₂₋₃ alkenyl; C₂₋₃ alkynyl; cyclopropyl; phenyl, which phenyl is optionally substituted by a group selected from halo, methyl, methoxy; pyridyl; and thiophenyl;
when there is a single bond present between C2' and C3',
R¹² is 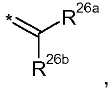 where R^{26a} and R^{26b} are independently selected from H, F, C₁₋₄ saturated alkyl, C₂₋₃ alkenyl, which alkyl and alkenyl groups are optionally substituted by a group selected from C₁₋₄ alkyl amido and C₁₋₄ alkyl ester; or, when one of R^{26a} and R^{26b} is H, the other is selected from nitrile and a C₁₋₄ alkyl ester;
R⁶ and R⁹ are independently selected from H, R, OH, OR, SH, SR, NH₂, NHR, NRR', nitro, Me₃Sn and halo;
where R and R' are independently selected from optionally substituted C₁₋₁₂ alkyl, C₃₋₂₀ heterocyclyl and C₅₋₂₀ aryl groups;
R⁷ is selected from H, R, OH, OR, SH, SR, NH₂, NHR, NHRR', nitro, Me₃Sn and halo;
R" is a C₃₋₁₂ alkylene group, which chain may be interrupted by one or more heteroatoms, e.g. O, S, NR^{N2} (where R^{N2} is H or C₁₋₄ alkyl), and/or aromatic rings, e.g. benzene or pyridine;
Y and Y' are selected from O, S, or NH;
R^{6'}, R^{7'}, R^{9'} are selected from the same groups as R⁶, R⁷ and R⁹ respectively;
   ***[Formula I]***
R^{L1'} is a linker for connection to the antibody (Ab);
R^{11a} is selected from OH, OR^{A}, where R^{A} is C₁₋₄ alkyl, and SO_{z}M, where z is 2 or 3 and M is a monovalent pharmaceutically acceptable cation;
R²⁰ and R²¹ either together form a double bond between the nitrogen and carbon atoms to which they are bound or;
R²⁰ is selected from H and R^{C}, where R^{C} is a capping group;
R²¹ is selected from OH, OR^{A} and SO_{z}M;
when there is a double bond present between C2 and C3, R² is selected from the group consisting of:
   (ia) C₅₋₁₀ aryl group, optionally substituted by one or more substituents selected from the group comprising: halo, nitro, cyano, ether, carboxy, ester, C₁₋₇ alkyl, C₃₋₇ heterocyclyl and bis-oxy-C₁₋₃ alkylene;
   (ib) C₁₋₅ saturated aliphatic alkyl;
   (ic) C₃₋₆ saturated cycloalkyl;
   (id) 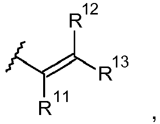 wherein each of R¹¹, R¹² and R¹³ are independently selected from H, C₁₋₃ saturated alkyl, C₂₋₃ alkenyl, C₂₋₃ alkynyl and cyclopropyl, where the total number of carbon atoms in the R² group is no more than 5;
   (ie) 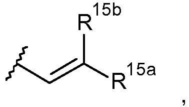 wherein one of R^{15a} and R^{15b} is H and the other is selected from: phenyl, which phenyl is optionally substituted by a group selected from halo, methyl, methoxy; pyridyl; and thiophenyl; and
   (if) 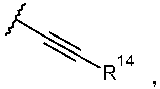 where R¹⁴ is selected from: H; C₁₋₃ saturated alkyl; C₂₋₃ alkenyl; C₂₋₃ alkynyl; cyclopropyl; phenyl, which phenyl is optionally substituted by a group selected from halo, methyl, methoxy; pyridyl; and thiophenyl;
when there is a single bond present between C2 and C3,
R² is 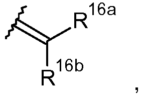 where R^{16a} and R^{16b} are independently selected from H, F, C₁₋₄ saturated alkyl, C₂₋₃ alkenyl, which alkyl and alkenyl groups are optionally substituted by a group selected from C₁₋₄ alkyl amido and C₁₋₄ alkyl ester; or, when one of R^{16a} and R^{16b} is H, the other is selected from nitrile and a C₁₋₄ alkyl ester;
***[Formula II]***
R²² is of formula Illa, formula IIIb or formula Illc:
   (a) 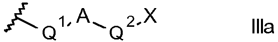 where A is a C₅₋₇ aryl group, and either
      (i) Q¹ is a single bond, and Q² is selected from a single bond and -Z-(CH₂)ₙ-, where Z is selected from a single bond, O, S and NH and n is from 1 to 3; or
      (ii) Q¹ is -CH=CH-, and Q² is a single bond;
   (b) 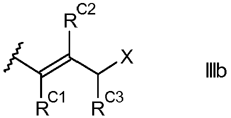 where;
      R^{C1}, R^{C2} and R^{C3} are independently selected from H and unsubstituted C₁₋₂ alkyl;
   (c) 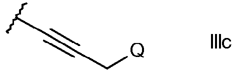 where Q is selected from O-R^{L2'}, S-R^{L2'} and NR^{N}-R^{L2'}, and R^{N} is selected from H, methyl and ethyl
      X is selected from the group comprising: O-R^{L2'}, S-R^{L2'}, CO₂-R^{L2'}, CO-R^{L2'}, NH-C(=O)-R^{L2'}, NHNH-R^{L2'}, CONHNH-R^{L2'}, 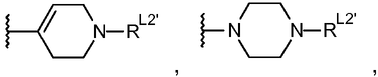 NR^{N}R^{L2'}, wherein R^{N} is selected from the group comprising H and C₁₋₄ alkyl;
      R^{L2'} is a linker for connection to the antibody (Ab);
      R¹⁰ and R¹¹ either together form a double bond between the nitrogen and carbon atoms to which they are bound or;
      R¹⁰ is H and R¹¹ is selected from OH, OR^{A} and SO_{z}M;
      R³⁰ and R³¹ either together form a double bond between the nitrogen and carbon atoms to which they are bound or;
      R³⁰ is H and R³¹ is selected from OH, OR^{A} and SO_{z}M.

In non-claimed aspects L-R^{L1'} or L-R^{L2'} is a group: 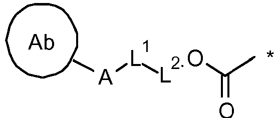 where the asterisk indicates the point of attachment to the PBD, Ab is the antibody, L¹ is a cleavable linker, A is a connecting group connecting L¹ to the antibody, L² is a covalent bond or together with -OC(=O)- forms a self-immolative linker.

In some of these non-claimed aspects, L¹ is enzyme cleavable.

It has previously been shown that such ADCs are useful in the treatment of CD19 expressing cancers (see, for example, WO2014/0571 17)**.**

In non-claimed aspects, the term anti-CD19-ADC may include any embodiment described in WO2014/057117. In particular, in non-claimed aspects the ADC may have the chemical structure: 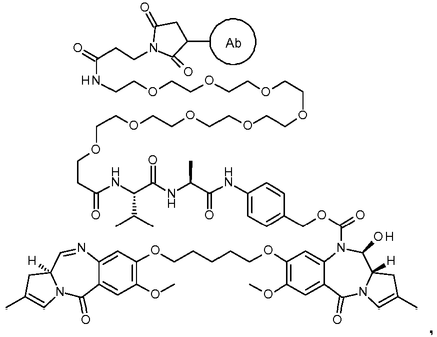 where the Ab is a CD19 antibody, and the DAR is between 1 and 8.

The antibody may comprise a VH domain having the sequence according to any one of SEQ ID NOs. 1, 2, 3, 4, 5 or 6, optionally further comprising a VL domain having the sequence according to any one of SEQ ID NOs. 7, 8, 9, 10, 11 or 12.

In some non-claimed aspects the antibody component of the anti-CD19-ADC is an antibody comprising: VH and VL domains respectively having the sequences of: SEQ ID NO. 1 and SEQ ID NO. 7, SEQ ID NO. 2 and SEQ ID NO. 8, SEQ ID NO. 3 and SEQ ID NO. 9, SEQ ID NO. 4 and SEQ ID NO. 10, SEQ ID NO. 5 and SEQ ID NO. 11, or SEQ ID NO. 6 and SEQ ID NO. 12.

In preferred non-claimed aspects the antibody comprises a VH domain having the sequence according to SEQ ID NO. 2. In preferred non-claimed aspects the antibody comprises a VL domain having the sequence according to SEQ ID NO. 8.

In the present invention the antibody comprises a VH domain and a VL domain, the VH and domain having the sequence of SEQ ID NO. 2 and the VL domain having the sequences of SEQ ID NO. 8.

The VH and VL domain(s) may pair so as to form an antibody antigen binding site that binds CD19.

In some embodiments the antibody is an intact antibody comprising a VH domain and a VL domain, the VH and VL domains having sequences of SEQ ID NO. 2 and SEQ ID NO. 8.

In some non-claimed aspects, the antibody is an antibody comprising a heavy chain having sequences of SEQ ID NO. 13 and a light chain having the sequences of SEQ ID NO. 14. In some embodiments the antibody is a fully human monoclonal IgG1 antibody, preferably IgG1, 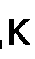 .

The antibody is the RB4v1.2 antibody described in WO2014/057117.

In an aspect the antibody is an antibody as described herein which has been modified (or further modified) as described below. In some embodiments the antibody is a humanised, deimmunised or resurfaced version of an antibody disclosed herein.

The anti-CD19-ADC for use with the aspects of the present invention is ADCx19, as described herein below.

In a non-claimed aspect, the anti-CD19-ADC for use with the aspects of the present disclosure is ADCT-402.

### ADCx19

ADCx19 is an antibody drug conjugate composed of a humanized antibody against human CD19 attached to a pyrrolobenzodiazepine (PBD) warhead via a cleavable linker. The mechanism of action of ADCX19 depends on CD19 binding. The CD19 specific antibody targets the antibody drug conjugate (ADC) to cells expressing CD19. Upon binding, the ADC internalizes and is transported to the lysosome, where the protease sensitive linker is cleaved and free PBD dimer is released inside the target cell. The released PBD dimer inhibits transcription in a sequence-selective manner, due either to direct inhibition of RNA polymerase or inhibition of the interaction of associated transcription factors. The PBD dimer produces covalent crosslinks that do not distort the DNA double helix and which are not recognized by nucleotide excision repair factors, allowing for a longer effective period (Hartley 2011).

It has the chemical structure:

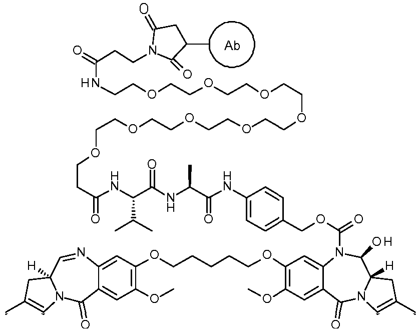

Ab represents Antibody RB4v1.2 (antibody with the VH and VL sequences SEQ ID NO. 2 and SEQ ID NO. 8, respectively). It may be synthesised as described in WO2014/057117 (RB4v1.2-E) and typically has a DAR (Drug to Antibody Ratio) of 2 +/-0.3.

### CD19 binding

As used herein, "binds CD19" is used to mean the antibody binds CD19 with a higher affinity than a non-specific partner such as Bovine Serum Albumin (BSA, Genbank accession no. CAA76847, version no. CAA76847.1 GI:3336842, record update date: Jan 7, 2011 02:30 PM). In some embodiments the antibody binds CD19 with an association constant (Kₐ) at least 2, 3, 4, 5, 10, 20, 50, 100, 200, 500, 1000, 2000, 5000, 10⁴, 10⁵ or 10⁶-fold higher than the antibody's association constant for BSA, when measured at physiological conditions. The antibodies can bind CD19 with a high affinity. For example, in some embodiments the antibody can bind CD19 with a K_{D} equal to or less than about 10⁻⁶ M, such as 1 x 10⁻⁶, 10⁻⁷, 10⁻⁸, 10⁻⁹,10⁻¹⁰, 10⁻¹¹, 10⁻¹², 10-¹³ or 10-14

In some embodiments, CD19 polypeptide corresponds to Genbank accession no.

NP_001171569, version no. NP_001171569.1 GI:296010921, record update date: Sep 10, 2012 12:43 AM. In one embodiment, the nucleic acid encoding CD19 polypeptide corresponds to Genbank accession no NM_001178098, version no. NM_001178098.1 GI:296010920, record update date: Sep 10, 2012 12:43 AM.In some embodiments, CD19 polypeptide corresponds to Uniprot/Swiss-Prot accession No. P15391.

### Anti-BCL-2 agents

Suitable non-claimed anti- BCL-2 agents include navitoclax (ABT-263), ABT-737, S55746/BCL201, and oblimersen (G3139). The anti-BCL-2 agent of the present invention is Venetoclax (ABT-199).

BCL-2 is localized to the outer membrane of mitochondria, where it plays an important role in promoting cellular survival and inhibiting the actions of pro-apoptotic proteins. The pro-apoptotic proteins in the BCL-2 family, including Bax and Bak, normally act on the mitochondrial membrane to promote permeabilization and release of cytochrome C and ROS, that are important signals in the apoptosis cascade. These pro-apoptotic proteins are in turn activated by BH3-only proteins, and are inhibited by the function of BCL-2 and its relative BCL-XI. The dynamic role of pro- and anti-apoptotic proteins, among other proteins, may alter the significance of increased BCL-2 expression in human disease. However, the wide variety of cancer types associated with aberrant expression of BCL-2 (both haematological and non-haematological solid tumours) is consistent with its role as an apoptotic regulator (see Hanada M., et al., Blood. 1993;82:1820-1828; Campos L., et al., Blood. 1993;81:3091-3096; Lamers F., et al., Eur J Cancer. 2012;48:3093-3103).

"Anti-BCL-2 agent" is used herein to mean any agent that specifically binds to and/or inhibits a biological activity of BCL-2. The term "the anti-BCL-2 agent" is used herein to mean the anti-BCL-2 of the present invention, i.e. venetoclax.

As used herein, "specifically binds BCL-2" is used to mean the agent binds BCL-2 with a higher affinity than a non-specific partner such as Bovine Serum Albumin (BSA, Genbank accession no. CAA76847, version no. CAA76847.1 GI:3336842, record update date: Jan 7, 2011 02:30 PM). In some embodiments the agent binds BCL-2 with an association constant (Kₐ) at least 2, 3, 4, 5, 10, 20, 50, 100, 200, 500, 1000, 2000, 5000, 10⁴, 10⁵ or 10⁶-fold higher than the agent's association constant for BSA, when measured at physiological conditions. The agents may bind BCL-2 with a high affinity. For example, in some embodiments the agent can bind BCL-2 with a K_{D} equal to or less than about 10⁻⁶ M, such as 1 x 10⁻⁶, 10⁻⁷, 10⁻⁸, 10⁻⁹,10⁻¹⁰, 10⁻¹¹, 10⁻¹², 10-¹³ or 10⁻¹⁴.

In some embodiments, BCL-2 polypeptide corresponds to Genbank accession no.

AAB72092, version no. AAB72092.1, record update date: Jul 24, 2016 02:22 PM. In one embodiment, the nucleic acid encoding BCL-2 polypeptide corresponds to Genbank accession no AF021792, version no. AF021792.1, record update date: Jul 24, 2016 02:22 PM. In some embodiments, BCL-2 polypeptide corresponds to Uniprot/Swiss-Prot accession No. Q92934.

To show that anti-CD19 ADCs works synergistically with the anti-BCL-2 agent, a panel of CD19 (+) cell lines will be co-treated with a range of concentration of both anti-CD19 ADC and the anti-BCL-2 agent. As negative controls, the same panel of cell lines will be treated with a range of concentrations of the anti-BCL-2 agent or with a range of concentration of anti-CD19 ADC and vehicle. After incubation, two parameters will be measured: the amount of surface CD19 (as determined by flow cytometry) and the in vitro cytotoxicity of the combinations (as determined by MTS assays). To determine the cytotoxicity, Cell viability is measured by adding MTS per well and incubating for 4 hours at 37°C. Percentage cell viability is calculated compared to the untreated control. Cytotoxic synergy is calculated by transforming the cell viability data into fraction affected, and calculating the combination index using the CalcuSyn analysis program.

Anti-BCL-2 include:
a) Venetoclax (ABT-199)
   i. CAS Number → 1257044-40-8
      (see http://www.cas.org/content/chemical-substances/faqs)
   ii. Drugbank reference → DB11581
      (see https://www.drugbank.ca/)
   iii. Unique Ingredient Identifier (UNII) → N54AIC43PW
      (see http://www.fda.gov/ForIndustry/DataStandards/SubstanceRegistrationSyst em-UniquelngredientldentifierUNII/default.htm) 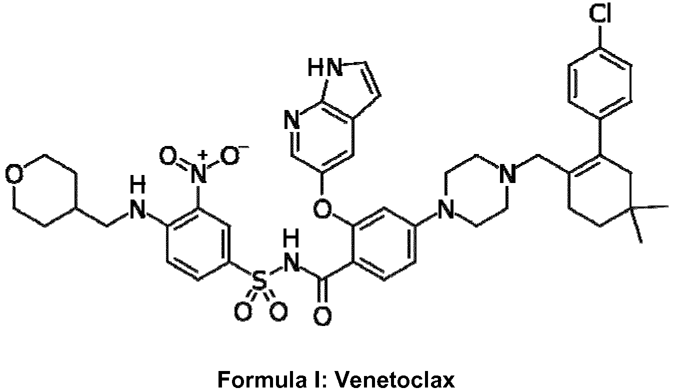
b) Navitoclax (ABT-263)
   i. CAS Number → 923564-51-6
      (see http://www.cas.org/content/chemical-substances/faqs)
   ii. Unique Ingredient Identifier (UNII) → XKJ5VVK2WD
      (see http://www.fda.gov/ForIndustry/DataStandards/SubstanceRegistrationSyst em-UniquelngredientldentifierUNII/default.htm) 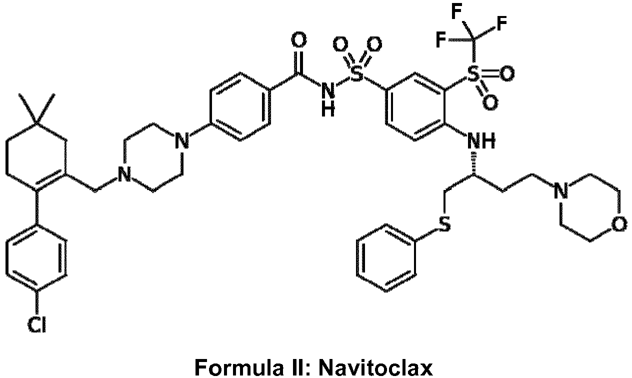
c) ABT-737
   i. CAS Number → 852808-04-9
      (see http://www.cas.org/content/chemical-substances/faqs)
   ii. Unique Ingredient Identifier (UNII) → Z5NFR173NV
      (see http://www.fda.gov/ForIndustry/DataStandards/SubstanceRegistrationSyst em-UniqueIngredientIdentifierUNII/default.htm) 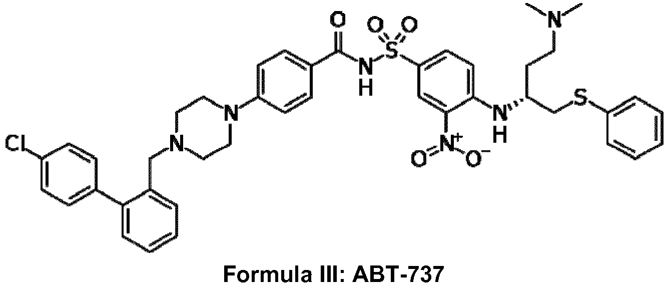
d) S55746/BCL201
   i. CAS Number → 1448584-12-0
      (see http://www.cas.org/content/chemical-substances/faqs) 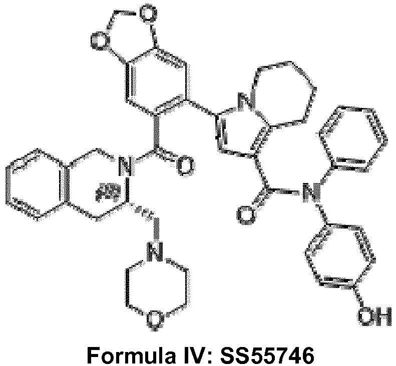
e) oblimersen (G3139)
   i. CAS Number → 190977-41-4
      (see http://www.cas.org/content/chemical-substances/faqs)
   ii. Drugbank reference → DB06650
      (see https://www.drugbank.ca/)
   iii. Unique Ingredient Identifier (UNII) → 85J5ZP6YSL
      (see http://www.fda.gov/ForIndustry/DataStandards/SubstanceRegistrationSyst em-UniqueIngredientIdentifierUNII/default.htm)
   iv. Oligo structure: D(P-thio)(T-C-T-C-C-C-A-G-C-G-T-G-C-G-C-C-A-T)

### Advantageous properties of the described combinations

Both the anti-CD19 ADC and anti-BCL-2 agent when used as a single agent in isolation have demonstrated clinical utility - for example, in the treatment of cancer. However, as described herein, combination of the anti-CD19 ADC and anti-BCL-2 agent is expected to provide one or more of the following advantages over treatment with either anti-CD19 ADC or anti-BCL-2 agent alone:
1) effective treatment of a broader range of cancers;
2) effective treatment of resistant or refractory forms of disorders such as cancer, and individuals with disorders such as cancer who have relapsed after a period of remission;
3) increased response rate to treatment; and / or
4) Increased durability of treatment.

Effective treatment of a broader range of cancers as used herein means that following treatment with the combination a complete response is observed with a greater range of recognised cancer types. That is, a complete response is seen from cancer types not previously reported to completely respond to either anti-CD19 ADC or anti-BCL-2 agent alone.

Effective treatment of a resistant, refractory, or relapsed forms as used herein means that following treatment with the combination a complete response is observed in individuals that are either partially or completely resistant or refractory to treatment with either anti-CD19 ADC or anti-BCL-2 agent alone (for example, individuals who show no response or only partial response following treatment with either agent alone, or those with relapsed disorder). In some embodiments, a complete response following treatment with the anti-CD19 ADC / anti-BCL-2 agent combination is observed at least 10% of individuals that are either partially or completely resistant or refractory to treatment with either anti-CD19 ADC or anti-BCL-2 agent alone. In some embodiments, a complete response following treatment with the anti-CD19 ADC / anti-BCL-2 agent combination is observed at least 20%, at least 30%, at least 40%, at least 50%, at least 60%, at least 70%, at least 80%, at least 90%, at least 95%, at least 98%, or at least 99% of individuals that are either partially or completely resistant or refractory to treatment with either anti-CD19 ADC or anti-BCL-2 agent alone.

Increased response rate to treatment as used herein means that following treatment with the combination a complete response is observed in a greater proportion of individuals than is observed following treatment with either anti-CD19 ADC or anti-BCL-2 agent alone. In some embodiments, a complete response following treatment with the anti-CD19 ADC / anti-BCL-2 agent combination is observed at least 10% of treated individuals. In some embodiments, a complete response following treatment with the anti-CD19 ADC / anti-BCL-2 agent combination is observed at least 20%, at least 30%, at least 40%, at least 50%, at least 60%, at least 70%, at least 80%, at least 90%, at least 95%, at least 98%, or at least 99% of treated individuals.

Increased durability of treatment as used herein means that average duration of complete response in individuals treated with the combination is longer than in individuals who achieve complete response following treatment with either anti-CD19 ADC or anti-BCL-2 agent alone. In some embodiments, the average duration of a complete response following treatment with the anti-CD19 ADC / anti-BCL-2 agent combination is at least 6 months. In some embodiments, the average duration of a complete response following treatment with the anti-CD19 ADC / anti-BCL-2 agent combination is at least 12 months, at least 18 months, at least 24 months, at least 3 years, at least 4 years, at least 5 years, at least 6 years, at least 7 years, at least 8 years, at least 9 years, at least 10 years, at least 15 years, or at least 20 years.

'Complete response' is used herein to mean the absence of any clinical evidence of disease in an individual. Evidence may be assessed using the appropriate methodology in the art, for example CT or PET scanning, or biopsy where appropriate. The number of doses required to achieve complete response may be one, two, three, four, five, ten or more. In some embodiments the individuals achieve complete response no more than a year after administration of the first dose, such as no more than 6 months, no more than 3 months, no more than a month, no more than a fortnight, or no more than a week after administration of the first dose.

### Treated disorders

The combined therapies described herein include those with utility for anticancer activity. In particular, in certain aspects the therapies include an antibody conjugated, i.e. covalently attached by a linker, to a PBD drug moiety, i.e. toxin. When the drug is not conjugated to an antibody, the PBD drug has a cytotoxic effect. The biological activity of the PBD drug moiety is thus modulated by conjugation to an antibody. The antibody-drug conjugates (ADC) of the disclosure selectively deliver an effective dose of a cytotoxic agent to tumor tissue whereby greater selectivity, i.e. a lower efficacious dose, may be achieved.

Thus, in one non-claimed aspect, the present disclosure provides combined therapies comprising administering an anti-CD19 ADC which binds CD19 for use in therapy, wherein the method comprises selecting a subject based on expression of the target protein.

In one aspect, the present disclosure provides the combined therapy with a label that specifies that the therapy is suitable for use with a subject determined to be suitable for such use. The label may specify that the therapy is suitable for use in a subject has expression of CD19, such as overexpression of CD19. The label may specify that the subject has a particular type of cancer.

The cancer may be lymphoma, such as non-Hodgkins lymphoma. The label may specify that the subject has a CD19+ lymphoma.

In a further non-claimed aspect there is also provided a combined therapy as described herein for use

in the treatment of a proliferative disease. Another non-claimed aspect of the present disclosure provides the use of a conjugate compound in the manufacture of a medicament for treating a proliferative disease.

One of ordinary skill in the art is readily able to determine whether or not a candidate combined therapy treats a proliferative condition for any particular cell type. For example, assays which may conveniently be used to assess the activity offered by a particular compound are described below.

The combined therapies described herein may be used to treat a proliferative disease.

The term "proliferative disease" pertains to an unwanted or uncontrolled cellular proliferation of excessive or abnormal cells which is undesired, such as, neoplastic or hyperplastic growth, whether *in vitro* or *in vivo.*

Examples of proliferative conditions include, but are not limited to, benign, pre-malignant, and malignant cellular proliferation, including but not limited to, neoplasms and tumours (e.g. histocytoma, glioma, astrocyoma, osteoma), cancers (e.g. lung cancer, small cell lung cancer, gastrointestinal cancer, bowel cancer, colon cancer, breast carinoma, ovarian carcinoma, prostate cancer, testicular cancer, liver cancer, kidney cancer, bladder cancer, pancreas cancer, brain cancer, sarcoma, osteosarcoma, Kaposi's sarcoma, melanoma), lymphomas, leukemias, psoriasis, bone diseases, fibroproliferative disorders (e.g. of connective tissues), and atherosclerosis. Cancers of interest include, but are not limited to, leukemias and ovarian cancers.

Any type of cell may be treated, including but not limited to, lung, gastrointestinal (including, e.g. bowel, colon), breast (mammary), ovarian, prostate, liver (hepatic), kidney (renal), bladder, pancreas, brain, and skin.

Proliferative disorders of particular interest include, but are not limited to, non-Hodgkin's Lymphoma, including diffuse large B-cell lymphoma (DLBCL), follicular lymphoma, (FL), Mantle Cell lymphoma (MCL), chronic lymphatic lymphoma (CLL), and Marginal Zone B-cell lymphoma (MZBL), and leukemias such as Hairy cell leukemia (HCL), Hairy cell leukemia variant (HCL-v), and Acute Lymphoblastic Leukaemia (ALL) such as Philadelphia chromosome-positive ALL (Ph+ALL) or Philadelphia chromosome-negative ALL (Ph-ALL). [Fielding A., Haematologica. 2010 Jan; 95(1): 8-12].

It is contemplated that the combined therapies of the present disclosure may be used to treat various diseases or disorders, e.g. characterized by the overexpression of a tumor antigen. Exemplary conditions or hyperproliferative disorders include benign or malignant tumors; leukemia, haematological, and lymphoid malignancies. Others include neuronal, glial, astrocytal, hypothalamic, glandular, macrophagal, epithelial, stromal, blastocoelic, inflammatory, angiogenic and immunologic, including autoimmune disorders and graft-versus-host disease (GVHD).

In the present invention, the disorder to be treated is cancer. Examples of cancer to be treated herein include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

In a non-claimed aspect, autoimmune diseases for which the combined therapies may be used in treatment include rheumatologic disorders (such as, for example, rheumatoid arthritis, Sjögren's syndrome, scleroderma, lupus such as SLE and lupus nephritis, polymyositis/dermatomyositis, cryoglobulinemia, anti-phospholipid antibody syndrome, and psoriatic arthritis), osteoarthritis, autoimmune gastrointestinal and liver disorders (such as, for example, inflammatory bowel diseases (e.g. ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-associated vasculitis, including Churg-Strauss vasculitis, Wegener's granulomatosis, and polyarteriitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, graft-versus-host disease (GVHD), and autoimmune endocrine disorders (such as, for example, diabetic-related autoimmune diseases such as insulin-dependent diabetes mellitus (IDDM), Addison's disease, and autoimmune thyroid disease (e.g. Graves' disease and thyroiditis)). More preferred such diseases include, for example, rheumatoid arthritis, ulcerative colitis, ANCA-associated vasculitis, lupus, multiple sclerosis, Sjögren's syndrome, Graves' disease, IDDM, pernicious anemia, thyroiditis, and glomerulonephritis.

In some aspects, the subject has a cancer selected from non-Hodgkin's Lymphoma, including diffuse large B-cell lymphoma (DLBCL), follicular lymphoma, (FL), Mantle Cell lymphoma (MCL), chronic lymphatic lymphoma (CLL), and Marginal Zone B-cell lymphoma (MZBL), and leukemias such as Hairy cell leukemia (HCL), Hairy cell leukemia variant (HCL-v), and Acute Lymphoblastic Leukaemia (ALL) such as Philadelphia chromosome-positive ALL (Ph+ALL) or Philadelphia chromosome-negative ALL (Ph-ALL). [Fielding A., Haematologica. 2010 Jan; 95(1): 8-12].

In certain aspects, the subject has diffuse large B cell lymphoma.

### Patient Selection

In certain aspects, the individuals are selected as suitable for treatment with the combined treatments before the treatments are administered.

As used herein, individuals who are considered suitable for treatment are those individuals who are expected to benefit from, or respond to, the treatment. Individuals may have, or be suspected of having, or be at risk of having cancer. Individuals may have received a diagnosis of cancer. In particular, individuals may have, or be suspected of having, or be at risk of having, lymphoma. In some cases, individuals may have, or be suspected of having, or be at risk of having, a solid cancer that has tumour associated non-tumor cells that express CD19, such as infiltrating cells that express CD19.

In some aspects, individuals are selected on the basis of the amount or pattern of expression of CD19. In some aspects, the selection is based on expression of CD19 at the cell surface.

In certain aspects, the target is BCL-2. In some aspects, the selection is based on expression of BCL-2.

In some aspects, the selection is based on levels of both CD19 at the cell surface and BCL-2.

In some cases, expression of the target in a particular tissue of interest is determined. For example, in a sample of lymphoid tissue or tumor tissue. In some cases, systemic expression of the target is determined. For example, in a sample of circulating fluid such as blood, plasma, serum or lymph.

In some aspects, the individual is selected as suitable for treatment due to the presence of target expression in a sample. In those cases, individuals without target expression may be considered not suitable for treatment.

In other aspects, the level of target expression is used to select a individual as suitable for treatment. Where the level of expression of the target is above a threshold level, the individual is determined to be suitable for treatment.

In some aspects, the presence of CD19 and/or in cells in the sample indicates that the individual is suitable for treatment with a combination comprising an anti-CD19 ADC and an anti-BCL-2 agent. In other aspects, the amount of CD19 and/or expression must be above a threshold level to indicate that the individual is suitable for treatment. In some aspects, the observation that CD19 and/or localisation is altered in the sample as compared to a control indicates that the individual is suitable for treatment.

In some aspects, an individual is indicated as suitable for treatment if cells obtained from lymph node or extra nodal sites react with antibodies against CD19 and/or as determined by IHC.

In some aspects, a patient is determined to be suitable for treatment if at least 5%, 10%, 15%, 20%, 25%, 30%, 35%, 40%, 45%, 50%, 55%, 60%, 65%, 70%, 75%, 80%, 85%, 90% or more of all cells in the sample express CD19. In some aspects disclosed herein, a patient is determined to be suitable for treatment if at least at least 10% of the cells in the sample express CD19.

In some aspects, a patient is determined to be suitable for treatment if at least 5%, 10%, 15%, 20%, 25%, 30%, 35%, 40%, 45%, 50%, 55%, 60%, 65%, 70%, 75%, 80%, 85%, 90% or more of all cells in the sample express. In some aspects disclosed herein, a patient is determined to be suitable for treatment if at least at least 10% of the cells in the sample express.

In some aspects, the individual is selected as suitable for treatment based on their current or previous treatment regime. In some embodiments the individual is selected for treatment with the anti-CD19 ADC if the individual has been treated with the anti-BCL-2 agent. In some embodiments the individual is selected for treatment with the anti-CD19 ADC if the individual is being treated with the anti-BCL-2 agent. In some cases the individual is selected for treatment if they are refractory to treatment (or further treatment) with the anti-BCL-2 agent. The anti-BCL-2 agent is Venetoclax. In embodiments where the individual is undergoing, or has undergone, treatment with the anti-BCL-2 agent, the anti-CD19 ADC may be administered in combination with the anti-BCL-2 agent, or without continued administration of the anti-BCL-2 agent.

In some embodiments the anti-CD19 ADC is administered to the selected individual in combination with the anti-BCL-2 agent. In some embodiments the anti-CD19 ADC is administered to the selected individual without continued administration of the anti-BCL-2 agent. The anti-BCL-2 agent is Venetoclax.

The term 'refractory to treatment (or further treatment) with the anti-BCL-2 agent' is used herein to mean that the disorder (such as cancer) does not respond, or has ceased to respond, to administration of the anti-BCL-2 agent when administered as a monotherapy. In some embodiments, individuals with refractory NHL are identified using the response criteria disclosed in Cheson at al., 2014 (South Asian J Cancer. 2014 Jan-Mar; 3(1): 66-70). In that document, non-responders are defined as individuals where there is either (i) a >50% increase from nadir in the sum product of diameters of any previously identified abnormal node, or (ii) an appearance of any new lesion during or at the end of therapy. In some embodiments, individuals with refractory leukaemia are identified as individuals with either stable or progressive disease who have completed one complete treatment cycle, or individual achieving partial response after two or more complete treatment cycles.

The term "is being treated with" is used herein to mean that the individual either has received the agent in question within the previous 3 weeks, or is scheduled to receive the agent in question within the next 3 weeks. In some embodiments, the term "is being treated with" is used herein to mean that the individual is receiving the agent in question in the same treatment cycle.

The term "has been treated with" is used herein to mean that the individual either has received the agent at any previous point. In some embodiments, the term "has been treated with" is used herein to mean that the individual has received the agent in question within the previous 12 months, for example, within the previous 3 months. In some embodiments, the term "has been treated with" is used herein to mean that the individual has received the agent in the immediately preceding treatment cycle.

### Samples

The sample may comprise or may be derived from: a quantity of blood; a quantity of serum derived from the individual's blood which may comprise the fluid portion of the blood obtained after removal of the fibrin clot and blood cells; a quantity of pancreatic juice; a tissue sample or biopsy; or cells isolated from said individual.

A sample may be taken from any tissue or bodily fluid. In certain aspects, the sample may include or may be derived from a tissue sample, biopsy, resection or isolated cells from said individual.

In certain aspects, the sample is a tissue sample. The sample may be a sample of tumor tissue, such as cancerous tumor tissue. The sample may have been obtained by a tumor biopsy. In some aspects, the sample is a lymphoid tissue sample, such as a lymphoid lesion sample or lymph node biopsy. In some cases, the sample is a skin biopsy.

In some aspects the sample is taken from a bodily fluid, more preferably one that circulates through the body. Accordingly, the sample may be a blood sample or lymph sample. In some cases, the sample is a urine sample or a saliva sample.

In some cases, the sample is a blood sample or blood-derived sample. The blood derived sample may be a selected fraction of a individual's blood, e.g. a selected cell-containing fraction or a plasma or serum fraction.

A selected cell-containing fraction may contain cell types of interest which may include white blood cells (WBC), particularly peripheral blood mononuclear cells (PBC) and/or granulocytes, and/or red blood cells (RBC). Accordingly, methods according to the present disclosure may involve detection of a first target polypeptide or nucleic acid in the blood, in white blood cells, peripheral blood mononuclear cells, granulocytes and/or red blood cells.

The sample may be fresh or archival. For example, archival tissue may be from the first diagnosis of an individual, or a biopsy at a relapse. In certain aspects, the sample is a fresh biopsy.

The first target polypeptide may be CD19.

### Individual status

The individual may be an animal, mammal, a placental mammal, a marsupial (e.g., kangaroo, wombat), a monotreme (e.g., duckbilled platypus), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orangutang, gibbon), or a human.

Furthermore, the individual may be any of its forms of development, for example, a foetus. In one preferred embodiment, the individual is a human. The terms "subject", "patient" and "individual" are used interchangeably herein.

In some aspects disclosed herein, an individual has, or is suspected as having, or has been identified as being at risk of, cancer. In some aspects disclosed herein, the individual has already received a diagnosis of cancer. The individual may have received a diagnosis of non-Hodgkin's Lymphoma, including diffuse large B-cell lymphoma (DLBCL), follicular lymphoma, (FL), Mantle Cell lymphoma (MCL), chronic lymphatic lymphoma (CLL), and Marginal Zone B-cell lymphoma (MZBL), and leukemias such as Hairy cell leukemia (HCL), Hairy cell leukemia variant (HCL-v), and Acute Lymphoblastic Leukaemia (ALL) such as Philadelphia chromosome-positive ALL (Ph+ALL) or Philadelphia chromosome-negative ALL (Ph-ALL). [Fielding A., Haematologica. 2010 Jan; 95(1): 8-12].

In some cases, the individual has received a diagnosis of non-Hodgkin's Lymphoma, including diffuse large B-cell lymphoma (DLBCL), follicular lymphoma, (FL), Mantle Cell lymphoma (MCL), chronic lymphatic lymphoma (CLL), and Marginal Zone B-cell lymphoma (MZBL), and leukemias such as Hairy cell leukemia (HCL), Hairy cell leukemia variant (HCL-v), and Acute Lymphoblastic Leukaemia (ALL) such as Philadelphia chromosome-positive ALL (Ph+ALL) or Philadelphia chromosome-negative ALL (Ph-ALL). [Fielding A., Haematologica. 2010 Jan; 95(1): 8-12].

In some cases, the individual has received a diagnosis of a solid cancer containing CD19+ expressing infiltrating cells.

The Individual may be undergoing, or have undergone, a therapeutic treatment for that cancer. The subject may, or may not, have previously received ADCX19. In some cases the cancer is lymphoma, including non-Hodgkins lymphoma.

The Individual may be undergoing, or have undergone, treatment with the anti-BCL-2 agent. In some cases the individual may be refractory to treatment (or further treatment) with the anti-BCL-2 The anti-BCL-2 agent is Venetoclax. In embodiments where the individual is undergoing, or has undergone, treatment with the anti-BCL-2 agent, the anti-CD19 ADC may be administered in combination with the anti-BCL-2 agent, or without continued administration of the anti-BCL-2 agent.

### Controls

In some aspects, target expression in the individual is compared to target expression in a control. Controls are useful to support the validity of staining, and to identify experimental artefacts.

In some cases, the control may be a reference sample or reference dataset. The reference may be a sample that has been previously obtained from a individual with a known degree of suitability. The reference may be a dataset obtained from analyzing a reference sample.

Controls may be positive controls in which the target molecule is known to be present, or expressed at high level, or negative controls in which the target molecule is known to be absent or expressed at low level.

Controls may be samples of tissue that are from individuals who are known to benefit from the treatment. The tissue may be of the same type as the sample being tested. For example, a sample of tumor tissue from a individual may be compared to a control sample of tumor tissue from a individual who is known to be suitable for the treatment, such as a individual who has previously responded to the treatment.

In some cases the control may be a sample obtained from the same individual as the test sample, but from a tissue known to be healthy. Thus, a sample of cancerous tissue from a individual may be compared to a non-cancerous tissue sample.

In some cases, the control is a cell culture sample.

In some cases, a test sample is analyzed prior to incubation with an antibody to determine the level of background staining inherent to that sample.

In some cases an isotype control is used. Isotype controls use an antibody of the same class as the target specific antibody, but are not immunoreactive with the sample. Such controls are useful for distinguishing non-specific interactions of the target specific antibody.

The methods may include hematopathologist interpretation of morphology and immunohistochemistry, to ensure accurate interpretation of test results. The method may involve confirmation that the pattern of expression correlates with the expected pattern. For example, where the amount of CD19 and/or BCL-2 expression is analyzed, the method may involve confirmation that in the test sample the expression is observed as membrane staining, with a cytoplasmic component. The method may involve confirmation that the ratio of target signal to noise is above a threshold level, thereby allowing clear discrimination between specific and non-specific background signals.

### Methods of Treatment

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included.

The term "therapeutically-effective amount" or "effective amount" as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen.

Similarly, the term "prophylactically-effective amount," as used herein, pertains to that amount of an active compound, or a material, composition or dosage from comprising an active compound, which is effective for producing some desired prophylactic effect, commensurate with a reasonable benefit/risk ratio, when administered in accordance with a desired treatment regimen.

Disclosed herein are methods of therapy. Also provided is a method of treatment of cancer, comprising administering to a subject in need of treatment a therapeutically-effective amount of the anti-CD19 ADC and the anti-BCL-2 agent. The term "therapeutically effective amount" is an amount sufficient to show benefit to a subject. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors. The subject may have been tested to determine their eligibility to receive the treatment according to the methods disclosed herein. The method of treatment may comprise a step of determining whether a subject is eligible for treatment, using a method disclosed herein.

The anti-CD19 ADC comprises an anti-CD19 antibody. The anti-CD19 antibody is RB4v1.2 antibody. The ADC comprises a drug which is a PBD dimer. The ADC is ADCx19. In non-claimed aspects, the ADC may be an ADC disclosed in WO2014/057117.

In non-claimed aspects, the anti-BCL-2 agent may be navitoclax (ABT-263), ABT-737, S55746/BCL201, and oblimersen (G3139). In the present invention, the anti-BCL-2 agent is Venetoclax (ABT-199).

The treatment may involve administration of the anti-CD19 ADC / anti-BCL-2 agent combination alone or in further combination with other treatments, either simultaneously or sequentially dependent upon the condition to be treated.

A non-claimed method of treatment involves:
(1) identifying an individual has been treated with, or is being treated with an anti-BCL-2 agent, such as Venetoclax;
(2) administering to the individual an anti-CD19 ADC, such as ADCx19; and, optionally
(3) administering to the individual an anti-BCL-2 agent, such as Venetoclax in combination with the anti-CD19 ADC (for example, at the same time as the ADC, or after the ADC).

Examples of treatments and therapies include, but are not limited to, chemotherapy (the administration of active agents, including, e.g. drugs, such as chemotherapeutics); surgery; and radiation therapy.

A "chemotherapeutic agent" is a chemical compound useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors. Chemotherapeutic agents include compounds used in "targeted therapy" and conventional chemotherapy.

Examples of chemotherapeutic agents include: Lenalidomide (REVLIMID^{®}, Celgene), Vorinostat (ZOLINZA^{®}, Merck), Panobinostat (FARYDAK^{®}, Novartis), Mocetinostat (MGCD0103), Everolimus (ZORTRESS^{®}, CERTICAN^{®}, Novartis), Bendamustine (TREAKISYM^{®}, RIBOMUSTIN^{®}, LEVACT^{®}, TREANDA^{®}, Mundipharma International), erlotinib (TARCEVA^{®}, Genentech/OSI Pharm.), docetaxel (TAXOTERE^{®}, Sanofi-Aventis), 5-FU (fluorouracil, 5-fluorouracil, CAS No. 51-21-8), gemcitabine (GEMZAR^{®}, Lilly), PD-0325901 (CAS No. 391210-10-9, Pfizer), cisplatin (cis-diamine, dichloroplatinum(II), CAS No. 15663-27-1), carboplatin (CAS No. 41575-94-4), paclitaxel (TAXOL^{®}, Bristol-Myers Squibb Oncology, Princeton, N.J.), trastuzumab (HERCEPTIN^{®}, Genentech), temozolomide (4-methyl-5-oxo- 2,3,4,6,8-pentazabicyclo [4.3.0] nona-2,7,9-triene- 9-carboxamide, CAS No. 85622-93-1, TEMODAR^{®}, TEMODAL^{®}, Schering Plough), tamoxifen ((Z)-2-[4-(1,2-diphenylbut-1-enyl)phenoxy]-N, N-dimethylethanamine, NOLVADEX^{®}, ISTUBAL^{®}, VALODEX^{®}), and doxorubicin (ADRIAMYCIN^{®}), Akti-1/2, HPPD, and rapamycin.

More examples of chemotherapeutic agents include: oxaliplatin (ELOXATIN^{®}, Sanofi), bortezomib (VELCADE^{®}, Millennium Pharm.), sutent (SUNITINIB^{®}, SU11248, Pfizer), letrozole (FEMARA^{®}, Novartis), imatinib mesylate (GLEEVEC^{®}, Novartis), XL-518 (Mek inhibitor, Exelixis, WO 2007/044515), ARRY-886 (Mek inhibitor, AZD6244, Array BioPharma, Astra Zeneca), SF-1126 (PI3K inhibitor, Semafore Pharmaceuticals), BEZ-235 (PI3K inhibitor, Novartis), XL-147 (PI3K inhibitor, Exelixis), PTK787/ZK 222584 (Novartis), fulvestrant (FASLODEX^{®}, AstraZeneca), leucovorin (folinic acid), rapamycin (sirolimus, RAPAMUNE^{®}, Wyeth), lapatinib (TYKERB^{®}, GSK572016, Glaxo Smith Kline), lonafarnib (SARASAR^{™}, SCH 66336, Schering Plough), sorafenib (NEXAVAR^{®}, BAY43-9006, Bayer Labs), gefitinib (IRESSA^{®}, AstraZeneca), irinotecan (CAMPTOSAR^{®}, CPT-11, Pfizer), tipifarnib (ZARNESTRA^{™}, Johnson & Johnson), ABRAXANE^{™} (Cremophor-free), albumin-engineered nanoparticle formulations of paclitaxel (American Pharmaceutical Partners, Schaumberg, II), vandetanib (rINN, ZD6474, ZACTIMA^{®}, AstraZeneca), chloranmbucil, AG1478, AG1571 (SU 5271; Sugen), temsirolimus (TORISEL^{®}, Wyeth), pazopanib (GlaxoSmithKline), canfosfamide (TELCYTA^{®}, Telik), thiotepa and cyclosphosphamide (CYTOXAN^{®}, NEOSAR^{®}); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g. calicheamicin, calicheamicin gamma1l, calicheamicin omegal1 (Angew Chem. Intl. Ed. Engl. (1994) 33:183-186); dynemicin, dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, morpholino-doxorubicin, cyanomorpholino-doxorubicin, 2-pyrrolino-doxorubicin and deoxydoxorubicin), epirubicin, esorubicin, idarubicin, nemorubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; 6-thioguanine; mercaptopurine; methotrexate; platinum analogs such as cisplatin and carboplatin; vinblastine; etoposide (VP-16); ifosfamide; mitoxantrone; vincristine; vinorelbine (NAVELBINE^{®}); novantrone; teniposide; edatrexate; daunomycin; aminopterin; capecitabine (XELODA^{®}, Roche); ibandronate; CPT-11; topoisomerase inhibitor RFS 2000; difluoromethylornithine (DMFO); retinoids such as retinoic acid; and pharmaceutically acceptable salts, acids and derivatives of any of the above. Combinations of agents may be used, such as CHP (doxorubicin, prednisone, cyclophosphamide), or CHOP (doxorubicin, prednisone, cyclophopsphamide, vincristine).

Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX^{®}; tamoxifen citrate), raloxifene, droloxifene, 4-hydroxytamoxifen, trioxifene, keoxifene, LY117018, onapristone, and FARESTON^{®} (toremifine citrate); (ii) aromatase inhibitors that inhibit the enzyme aromatase, which regulates estrogen production in the adrenal glands, such as, for example, 4(5)-imidazoles, aminoglutethimide, MEGASE^{®} (megestrol acetate), AROMASIN^{®} (exemestane; Pfizer), formestanie, fadrozole, RIVISOR^{®} (vorozole), FEMARA^{®} (letrozole; Novartis), and ARIMIDEX^{®} (anastrozole; AstraZeneca); (iii) anti-androgens such as flutamide, nilutamide, bicalutamide, leuprolide, and goserelin; as well as troxacitabine (a 1,3-dioxolane nucleoside cytosine analog); (iv) protein kinase inhibitors such as MEK inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE^{®}, Genta Inc.); (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAXID^{®}; PROLEUKIN^{®} rIL-2; topoisomerase 1 inhibitors such as LURTOTECAN^{®}; ABARELIX^{®} rmRH; (ix) anti-angiogenic agents such as bevacizumab (AVASTIN^{®}, Genentech); and pharmaceutically acceptable salts, acids and derivatives of any of the above.

Also included in the definition of "chemotherapeutic agent" are therapeutic antibodies such as alemtuzumab (Campath), bevacizumab (AVASTIN^{®}, Genentech); cetuximab (ERBITUX^{®}, Imclone); panitumumab (VECTIBIX^{®}, Amgen), pertuzumab (PERJETA^{™}, OMNITARG^{™}, 2C4, Genentech), trastuzumab (HERCEPTIN^{®}, Genentech), MDX-060 (Medarex) and the antibody drug conjugate, gemtuzumab ozogamicin (MYLOTARG^{®}, Wyeth).

Humanized monoclonal antibodies with therapeutic potential as chemotherapeutic agents in combination with the conjugates of the disclosure include: alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

Compositions according to the present disclosure are preferably pharmaceutical compositions. Pharmaceutical compositions according to the present disclosure, and for use in accordance with the present disclosure, may comprise, in addition to the active ingredient, i.e. a conjugate compound, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous, or intravenous.

Pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

### Dosage

It will be appreciated by one of skill in the art that appropriate dosages of the anti-CD19 ADC and the anti-BCL-2 agent, and compositions comprising these active elements, can vary from subject to subject. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the subject. The amount of compound and route of administration will ultimately be at the discretion of the physician, veterinarian, or clinician, although generally the dosage will be selected to achieve local concentrations at the site of action which achieve the desired effect without causing substantial harmful or deleterious side-effects.

In certain aspects, the dosage of anti-CD19 ADC is determined by the expression of CD19 observed in a sample obtained from the subject. Thus, the level or localisation of expression of CD19 in the sample may be indicative that a higher or lower dose of anti-CD19 ADC is required. For example, a high expression level of CD19 may indicate that a higher dose of anti-CD19 ADC would be suitable. In some cases, a high expression level of CD19 may indicate the need for administration of another agent in addition to the anti-CD19 ADC. For example, administration of the anti-CD19 ADC in conjunction with a chemotherapeutic agent. A high expression level of CD19 may indicate a more aggressive therapy.

In certain aspects, the dosage of the anti-BCL-2 agent is determined by the expression of observed in a sample obtained from the subject. Thus, the level or localisation of expression of in the sample may be indicative that a higher or lower dose of anti-BCL-2 agent is required. For example, a high expression level of BCL-2 may indicate that a higher dose of anti-BCL-2 agent would be suitable. In some cases, a high expression level of BCL-2 may indicate the need for administration of another agent in addition to the anti-BCL-2 agent. For example, administration of the anti-BCL-2 agent in conjunction with a chemotherapeutic agent. A high expression level of BCL-2 may indicate a more aggressive therapy.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of each active compound is in the range of about 100 ng to about 25 mg (more typically about 1 µg to about 10 mg) per kilogram body weight of the subject per day. Where the active compound is a salt, an ester, an amide, a prodrug, or the like, the amount administered is calculated on the basis of the parent compound and so the actual weight to be used is increased proportionately.

In one embodiment, each active compound is administered to a human subject according to the following dosage regime: about 100 mg, 3 times daily.

In one embodiment, each active compound is administered to a human subject according to the following dosage regime: about 150 mg, 2 times daily.

In one embodiment, each active compound is administered to a human subject according to the following dosage regime: about 200 mg, 2 times daily.

However in one embodiment, each conjugate compound is administered to a human subject according to the following dosage regime: about 50 or about 75 mg, 3 or 4 times daily.

In one embodiment, each conjugate compound is administered to a human subject according to the following dosage regime: about 100 or about 125 mg, 2 times daily.

For the anti-CD19 ADC, the dosage amounts described above may apply to the conjugate (including the PBD moiety and the linker to the antibody) or to the effective amount of PBD compound provided, for example the amount of compound that is releasable after cleavage of the linker.

The anti-CD19 ADC is ADCx19.

The anti-BCL-2 agent is Venetoclax (ABT-199).

### Antibodies

The term "antibody" herein is used in the broadest sense and specifically covers monoclonal antibodies, polyclonal antibodies, dimers, multimers, multispecific antibodies (*e.g.,* bispecific antibodies), intact antibodies (also described as "full-length" antibodies) and antibody fragments, so long as they exhibit the desired biological activity, for example, the ability to bind CD19 (Miller et al (2003) Jour. of Immunology 170:4854-4861). Antibodies may be murine, human, humanized, chimeric, or derived from other species such as rabbit, goat, sheep, horse or camel.

An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by Complementarity Determining Regions (CDRs) on multiple antibodies. Each antibody that specifically binds to a different epitope has a different structure. Thus, one antigen may have more than one corresponding antibody. An antibody may comprise a full-length immunoglobulin molecule or an immunologically active portion of a full-length immunoglobulin molecule, *i.e.,* a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD, and IgA), class (e.g. IgG1, IgG2, IgG3, IgG4, IgA1 and IgA2) or subclass, or allotype (e.g. human G1m1, G1m2, G1m3, non-G1m1 [that, is any allotype other than G1m1], G1m17, G2m23, G3m21, G3m28, G3m11, G3m5, G3m13, G3m14, G3m10, G3m15, G3m16, G3m6, G3m24, G3m26, G3m27, A2m1, A2m2, Km1, Km2 and Km3) of immunoglobulin molecule. The immunoglobulins can be derived from any species, including human, murine, or rabbit origin.

"Antibody fragments" comprise a portion of a full length antibody, generally the antigen binding or variable region thereof. Examples of antibody fragments include Fab, Fab', F(ab')₂, and scFv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al (1975) Nature 256:495, or may be made by recombinant DNA methods (see, US 4816567). The monoclonal antibodies may also be isolated from phage antibody libraries using the techniques described in Clackson et al (1991) Nature, 352:624-628; Marks et al (1991) J. Mol. Biol., 222:581-597 or from transgenic mice carrying a fully human immunoglobulin system (Lonberg (2008) Curr. Opinion 20(4):450-459).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4816567; and Morrison et al (1984) Proc. Natl. Acad. Sci. USA, 81:6851-6855). Chimeric antibodies include "primatized" antibodies comprising variable domain antigen-binding sequences derived from a non-human primate (e.g. Old World Monkey or Ape) and human constant region sequences.

An "intact antibody" herein is one comprising VL and VH domains, as well as a light chain constant domain (CL) and heavy chain constant domains, CH1, CH2 and CH3. The constant domains may be native sequence constant domains (e.g. human native sequence constant domains) or amino acid sequence variant thereof. The intact antibody may have one or more "effector functions" which refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; and down regulation of cell surface receptors such as B cell receptor and BCR.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

### Brief Description of the Figures

Embodiments and experiments illustrating the principles of the disclosure will now be discussed with reference to the accompanying figures in which:

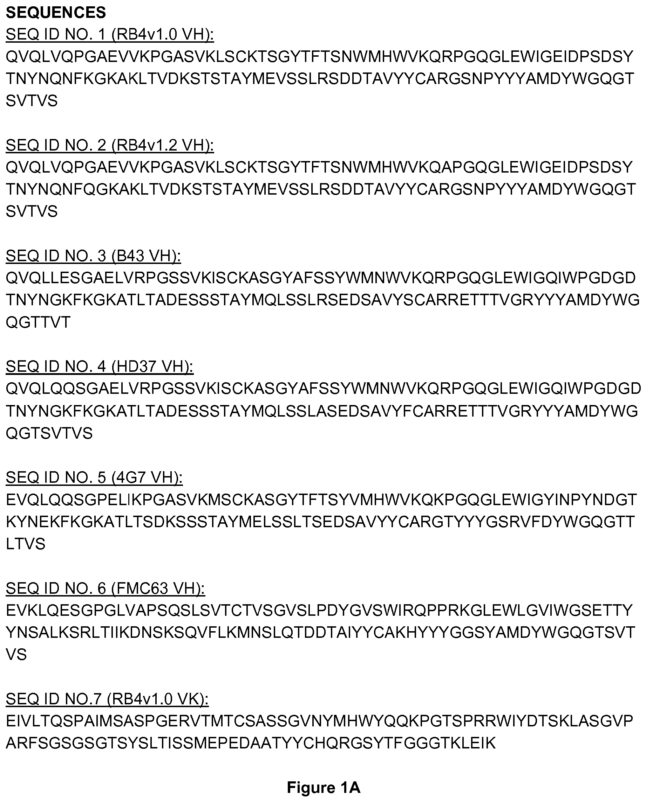

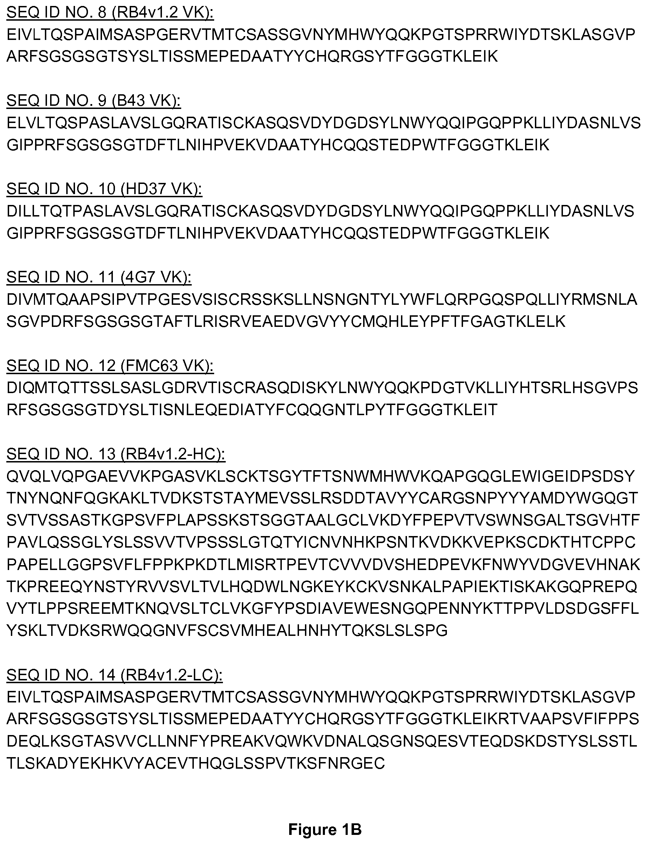

### Figure 1. Sequences

The disclosure includes the combination of the aspects and preferred features described except where such a combination is clearly impermissible or expressly avoided.

The section headings used herein are for organizational purposes only and are not to be construed as limiting the subject matter described.

Aspects and embodiments of the present disclosure will now be illustrated, by way of example, with reference to the accompanying figures. Further aspects and embodiments will be apparent to those skilled in the art.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

### EXAMPLES

In the following examples:
- Cell lines expressing CD19 suitable for use in the examples include Ramos, Daudi, Raji, WSU-DLCL and NALM-6 cells.
- Disease A - Diffuse Large B-cell Lymphoma/DLBC is an aggressive type of non-Hodgkin lymphoma that develops from the B-cells in the lymphatic system. It constitutes the largest subgroup of non-Hodgkin lymphoma.
- Disease B - Mantle Cell Lymphoma/MCL is a rare B-cell NHL that most often affects men over the age of 60. The disease may be aggressive (fast growing) but it can also behave in a more indolent (slow growing) fashion in some patients. MCL comprises about five percent of all NHLs.
- Disease C - Follicular lymphoma/FL is a fairly indolent type of NHL with long survival time but for which it is very difficult to achieve a cure; it can also transform into more aggressive forms of lymphoma.

### Example 1 (Reference example)

In a separate experiment, cell lines expressing CD19 will be incubated for 0, 6, 24 and 48 hours with etoposide (negative control) and oxaliplatin (positive control), 1 µg/mL anti-CD19 ADC (ADC targeting CD19 with a PBD dimer warhead), 1 µg/mL anti-CD19 (the antibody in ADC) and 1 µg/mL of B12-SG3249 (a non-binding control ADC with the same PBD payload as anti-CD19 ADC).

After incubation, the cells are washed, and fed to human Dendritic cells (DCs) for an additional 24 h. Activation of the DCs is subsequently measured by increased surface expression of CD86 on the DC population (as determined by Flow cytometry) and by measuring DC mediated release of IL-8 and MIP2.

### Example 2 (Reference example)

The purpose of this study is to preliminarily assess the safety, tolerability, pharmacological and clinical activity of this combination

The following cancer types have been chosen for study: Disease A, Disease B, and Disease C

Evidence for efficacy as single agents exists for both drugs:
- anti CD19 ADC (see, for example, WO2014/057117, WO2016/166298, WO2014/057122, and WO2016/166307)
- Anti-BCL-2 agent (see KS Peggs et al.2009, Clinical and Experimental Immunology, 157: 9-19 [doi:10.1111/j.1365-2249.2009.03912.x])

This primary purpose of this study is to explore whether these agents can be safely combined, and if so, will identify the dose(s) and regimens appropriate for further study. The study will also assess whether each combination induces pharmacologic changes in tumor that would suggest potential clinical benefit.

In addition, it will provide preliminary evidence that a combination may increase the response rate and durability of response compared with published data for treatment with single agent anti-CD19 ADC or anti-BCL-2 agent.

Each disease group may include a subset of patients previously treated with the anti-BCL-2 agent to explore whether combination therapy might overcome resistance to anti-BCL-2 agent therapy. For each disease, it is not intended to apply specific molecular selection as the data available at present generally do not support excluding patients on the basis of approved molecular diagnostic tests.

### Rationale for anti CD19 ADC starting dose

The RDE for already established for ADC (in ug/kg administered every three weeks) will be used for all patients in this study. To ensure patient safety, a starting dose below the RDE will be used; the starting dose level will be one where patient benefit could still be demonstrated in study ADC1, suggesting that patients enrolled at such dose level will gain at least some benefit by taking part.

### Rationale for anti-BCL-2 agent starting dose

The RDE for already established for the anti-BCL-2 agent (in ug/kg administered every three weeks) will be used for all patients in this study. To ensure patient safety, a starting dose below the RDE will be used; the starting dose level will be one where patient benefit could still be demonstrated in study SA1, suggesting that patients enrolled at such dose level will gain at least some benefit by taking part.

### Objectives and related endpoints

| **Objective** | **Endpoint** |
|---|---|
| *Primary Objective* | Frequency and severity of treatment-emergent AEs and SAEs |
| To characterize the safety and tolerability of ADC in combination with the anti-BCL-2 agent, and to identify the recommended dose and schedules for future studies | |
| | Changes between baseline and post-baseline laboratory parameters and vital signs |
| | Incidence of dose limiting toxicities (DLTs) during the first cycle of treatment (dose escalation only) |
| | Frequency of dose interruptions and dose reductions |
| *Secondary Objectives* | ORR, DOR, PFS, OS |
| To evaluate the clinical activity of the combination of ADC with the anti-BCL-2 agent | |
| | AUC and Cmax for each compound |
| To characterize the pharmacokinetic (PK) profile of each of the two compounds ADC and the anti-BCL-2 agent | |
| Evidence for immunogenicity and ADAs to ADC | Anti-Drug-Antibodies (ADAs) before, during and after treatment with ADC |
| *Exploratory Objectives* | |
| To examine potential correlation of PK profiles with safety/tolerability and efficacy | Correlation coefficients between AUC and/or Cmax of each compound or a compound measure and any of the safety or efficacy variables |
| To characterize changes in the immune infiltrate in tumors | Immunohistochemistry of pre- and on-treatment tumor biopsies, |
| To characterize changes in circulating levels of cytokines in plasma and markers of activation in circulating immune cells | Measurements (e.g. via ELISA) of immunologically relevant cytokines in plasma or serum; staining levels for activation markers of circulating immune cells (e.g. FACS) |

### Study design

This phase Ib, multi-center, open-label study to characterize the safety, tolerability, pharmacokinetics (PK), pharmacodynamics (PD) and antitumor activity of the ADC in combination with the anti-BCL-2 agent, in patients with disease A, disease B, and disease C.

The study is comprised of a dose escalation part followed by a dose expansion part.

Dose escalation will start with reduced starting doses (compared to their respective recommended phase 2 or licensed dose levels), for both ADC and the anti-BCL-2 agent, to guarantee patient safety. Starting doses will be 33% (or 50%) of the RDE for each compound. Subsequently, doses will be first escalated for the anti-BCL-2 agent until the RDE or licensed dose has been reached, or a lower dose if necessary for tolerability reasons. Then, the dose for ADC will be escalated, until the RDE for combination treatment is reached. This is visualized in the below diagram:

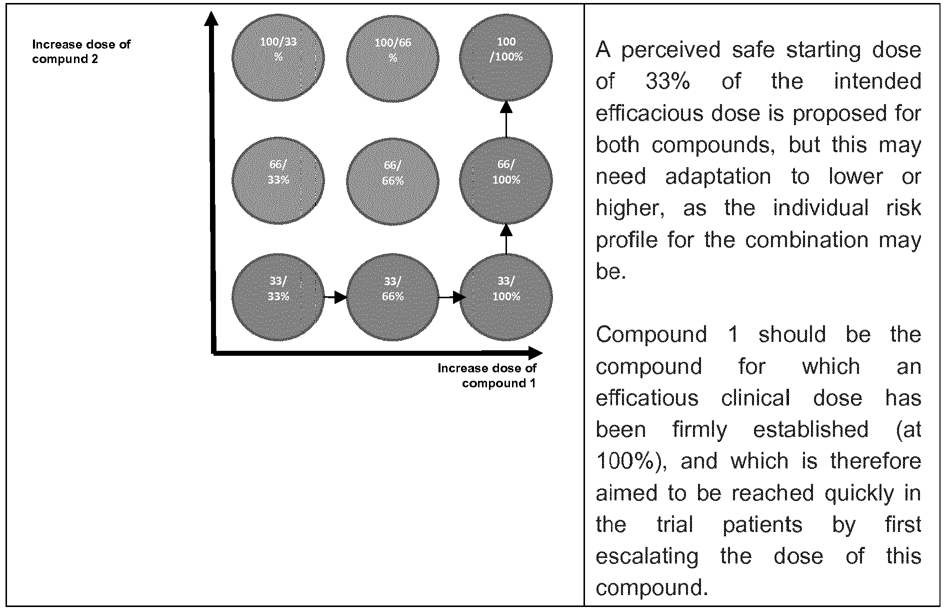
Increase dose of
compund 2
100/33 | 100/66 | 100
% | % | /100%
66/ | 66/ | 66/
33% | 66% | 100%
33/ | 33/ | 33/
33% | 66% | 100%
Increase dose of
compound 1
|
A perceived safe starting dose
of 33% of the intended
efficacious dose is proposed for
both compounds, but this may
need adaptation to lower or
higher, as the individual risk
profile for the combination may
be.

Compound 1 should be the
compound for which an
efficacious clinical dose has
been firmly established (at
100%), and which is therefore
aimed to be reached quickly in
the trial patients by first
escalating the dose of this
compound.

If the dose combination is determined to be safe, it may be tested in additional patients to confirm the safety and tolerability at that dose level. Further tailoring of the dose of each compound may be conducted, and/or the regimen may be modified.

The dose escalation of the combination will be guided by a Bayesian Logistic Regression Model (BLRM) based on any Dose Limiting Toxicities (DLTs) observed in the first (or first two, TBC) cycles of therapy. Use of a BLRM is a well-established method to estimate the maximum tolerated dose (MTD)/ recommended dose for expansion (RDE) in cancer patients. The adaptive BLRM will be guided by the Escalation With Overdose Control (EWOC) principle to control the risk of DLT in future patients on the study. The use of Bayesian response adaptive models for small datasets has been accepted by FDA and EMEA ("Guideline on clinical trials in small populations", February 1, 2007) and endorsed by numerous publications (Babb et al. 1998, Neuenschwander et al. 2008).

The decisions on new dose combinations are made by the Investigators and sponsor study personnel in a dose escalation safety call (DESC) based upon the review of patient tolerability and safety information (including the BLRM summaries of DLT risk, if applicable) along with PK, PD and preliminary activity information available at the time of the decision.

Once the MTD(s)/RDE is determined for the combination, the expansion part of the study may be initiated to further assess the safety, tolerability and preliminary efficacy.
■ For combinations with IO, changes in the immune infiltrate in tumors will also be characterized following combination treatment in the target disease indications.

Given the available prior clinical experience with the agents in this study, it is expected that in most cases a combination dose can be identified without testing a large number of dose levels or schedules. To assess the pharmacodynamic activity of the combinations, patients will be asked to undergo a tumor biopsy at baseline and again after approximately two cycles of therapy.
■ For IO combo: The extent of the change in tumor infiltration by immune cells including lymphocytes and macrophages will contribute to a decision on any potential benefit.

### Dose escalation part

During the dose escalation part of the study, patients will be treated with a fixed dose of ADC administered i.v., and increasing doses of the anti-BCL-2 agent until the RDE for the anti-BCL-2 agent has been reached. Subsequently, doses of ADC are increased (in different cohorts) while the dose for the anti-BCL-2 agent is kept constant.

Two to approximately 3 or 4 patients with disease A, disease B or disease C will be treated in each escalation cohort until the determination of MTD(s)/RDE(s) is determined.

There will be a 24-hour observation before enrolling the second patient at Dose Level 1. The DLT observation period at each dose level is either 1 cycle (3 weeks) or 2 cycles (6 weeks) as mandated by the appropriate authorities for IO therapies, after which it will be determined whether to escalate to the next dose level, stay at the current dose level, or de-escalate to the previous dose level for the next cohort. There will be no de-escalation from Dose Level 1. Intrapatient dose escalation is not permitted.

Dose escalation is not permitted unless 2 or more patients have complete DLT information through the first cycle in any given dose level. Dose escalation will be determined by using a mCRM with a target DLT rate of 30% and an equivalence interval of 20% to 35%, and with dose escalation-with-overdose-control (EWOC) and no dose skipping.

Patients will be assigned to a cohort that is actively enrolling. Dose escalation will be performed in each combination following the completion of one cycle of treatment. Safety assessments including adverse events (AEs) and laboratory values will be closely monitored for all enrolled patients in order to identify any DLTs. A single MTD/RDE will be defined; a disease-specific MTD/RDE will not be established.

The mCRM will be implemented for DE under the oversight of a Dose Escalation Steering Committee (DESC). The DESC will confirm each escalating dose level after reviewing all available safety data. PK data from patients in that dose level and prior dose levels may also inform decision making. The DESC may halt dose escalation prior to determining the MTD based on emerging PK, PD, toxicity or response data.

Additional patients may be included at any dose level to further assess the safety and tolerability if at least 1 patient in the study has achieved a partial response or better, or if further evaluation of PK or PD data is deemed necessary by the DESC to determine the RDE.

Dose Escalation will be stopped after 3 cohorts (or at least 6 patients) are consecutively assigned to the same dose level. If the MTD is not reached, the recommended dose for expansion (RDE) will be determined. Prior to the determination of the MTD/RDE a minimum of 6 patients must have been treated with the combination.

It is intended that paired tumor biopsies will be obtained from patients during dose escalation. Analysis of these biopsies will contribute to a better understanding of the relationship between the dose and the pharmacodynamic activity of the combination.

### Safety Oversight by the Dose Escalation Steering Committee

A DESC comprised of ADC Therapeutics and the investigators will review patient safety on an ongoing basis during the DE to determine if the dose escalation schedule prescribed by the mCRM warrants modification. In addition to safety observations, PK and/or PD data may also inform decision making. Intermediate doses may be assigned after agreement between ADC Therapeutics and investigators. The DESC may continue to provide oversight during Part 2. No formal Data Safety Monitoring Board (DSMB) will be used.

### Dose expansion part

Once the MTD/RDE has been declared, dose expansion part may begin. The main objective of the expansion part is to further assess the safety and tolerability of the study treatment at the MTD/RDE and to gain a preliminary understanding of the efficacy of the combination compared to historical single agent efficacy data.

An important exploratory objective is to assess changes in the immune infiltrate in tumor in response to treatment. This will be assessed in paired tumor biopsies collected from patients, with a minimum of ten evaluable biopsy pairs (biopsy specimens must contain sufficient tumor for analysis) in patients treated at the MTD/RDE. If this is not feasible, collection of these biopsies may be stopped. A minimum of 10 to 20 patients are planned to be treated in each investigational arm,

Several different investigational arms will open, one per disease. A total of nine investigational arms may be run in the dose expansion. Should enrollment for any of these groups not be feasible, then enrollment to that group may be closed before the 10 to 20 patients target is met.

In each treatment group a maximum of approximately six patients who have received and progressed on prior single administration (i.e. not in combination) anti-BCL-2 agent therapy will be allowed to be treated. This number may be increased if a combination shows promise of overcoming resistance to prior treatment with single administration anti-BCL-2 agent.

### Patient Population

The study will be conducted in adult patients with advanced Disease A, Disease B or Disease C as outlined above. The investigator or designee must ensure that only patients who meet all the following inclusion and none of the exclusion criteria are offered treatment in the study.

### Inclusion criteria

Patients eligible for inclusion in this study have to meet all of the following criteria:
1. Written informed consent must be obtained prior to any procedures
2. Age 18
   years.
3. Patients with advanced/metastatic cancer, with measurable disease as determined by RECIST version 1.1, who have progressed despite standard therapy or are intolerant to standard therapy, or for whom no standard therapy exists. Patients must fit into one of the following groups:
   - Disease A
   - Disease B
   - Disease C
4. ECOG Performance Status 0 - 1 (or 2 TBC)
5. TBC: Patient must have a site of disease amenable to biopsy, and be a candidate for tumor biopsy according to the treating institution's guidelines. Patient must be willing to undergo a new tumor biopsy at baseline, and again during therapy on this study.
6. Prior therapy with the anti-BCL-2 agent or related compounds (i.e. same MOA) is allowed

### Exclusion criteria

Patients eligible for this study must not meet any of the following criteria:
1. History of severe hypersensitivity reactions to other mAbs (OR to same backbone mAb as in ADC OR to same IO mAb if applicable)
2. Known history of positive serum human ADA to backbone of mAb as in ADC
3. Central Nervous System (CNS) disease only (if applicable)
4. Symptomatic CNS metastases or evidence of leptomeningeal disease (brain MRI or previously documented cerebrospinal fluid (CSF) cytology)
   ➢ Previously treated asymptomatic CNS metastases are permitted provided that the last treatment (systemic anticancer therapy and-or local radiotherapy) was completed >= 8 weeks prior to 1^{st} day of dosing, except usage of low dose steroids on a taper is allowed)
   ➢ Patients with discrete dural metastases are eligible.
5. Patient having out of range laboratory values defined as:
   - Serum creatinine <= 1.5 x ULN. If serum creatinine > 1.5, the creatinine clearance (calculated using Cockcroft-Gault formula, or measured) must be > 60 mL/min/1.73m2 for a patient to be eligible
   - Total bilirubin > 1.5 x ULN, except for patients with Gilbert's syndrome who are excluded if total bilirubin > 3.0 x ULN or direct bilirubin > 1.5 x ULN
   - Alanine aminotransferase (ALT) > 3 x ULN, except for patients that have tumor involvement of the liver, who are excluded if ALT > 5 x ULN
   - Aspartate aminotransferase (AST) > 3 x ULN, except for patients that have tumor involvement of the liver, who are excluded if AST > 5 x ULN
   - Absolute neutrophil count< 1.0 x 10e9/L
   - Platelet count< 75 x 10e9/L
   - Hemoglobin (Hgb) < 8 g/dL
   - Potassium, magnesium, calcium or phosphate abnormality > CTCAE grade 1 despite appropriate replacement therapy
6. Impaired cardiac function or clinically significant cardiac disease, including any of the following:
   - Clinically significant and/or uncontrolled heart disease such as congestive heart failure requiring treatment (NYHA grade III or IV) or uncontrolled hypertension defined by a Systolic Blood Pressure (SBP) 160 mm Hg and/or Diastolic Blood Pressure (DBP) 100 mm Hg, with or without anti-hypertensive medication.
   - QTcF >470 msec for females or >450 msec for males on screening ECG using Fridericia's correction, congenital long QT syndrome
   - Acute myocardial infarction or unstable angina pectoris < 3 months (months prior to study entry
   - Clinically significant valvualr disease with documented compromise in cardiac function
   - Symptomatic pericarditis
   - History of or ongoing documented cardiomyopathy
   - Left Ventricular Ejection Fraction (LVEF) <40%, as determined by echocardiogram (ECHO) or Multi gated acquisition (MUGA) scan
   - History or presence of any clinically significant cardiac arrhythmias, e.g. ventricular, supraventricular, nodal arrhythmias, or conduction abnormality (TBC qualifier: ... requiring a pacemaker or not controlled with medication)
   - Presence of unstable atrial fibrillation (ventricular response rate> 100 bpm).
      ➢ NOTE: Patients with stable atrial fibrillation can be enrolled provided they do not meet other cardiac exclusion criteria.
   - Complete left bundle branch block (LBBB), bifascicular block
   - Any clinically significant ST segment and/or T-wave abnormalities
7. Toxicity attributed to prior IO therapy that led to discontinuation of therapy. Adequately treated patients for drug-related skin rash or with replacement therapy for endocrinopathies are not excluded, provided these toxicities did not lead to the discontinuation of prior treatment.
8. Patients with active, known or suspected autoimmune disease. Subjects with vitiligo, type I diabetes mellitus, residual hypothyroidism due to autoimmune condition only requiring hormone replacement, psoriasis not requiring systemic treatment, or conditions not expected to recur in the absence of an external trigger are permitted to enroll, provided the trigger can be avoided.
9. Human Immunodeficiency Virus (HIV), or active Hepatitis B (HBV) or Hepatitis C (HCV) virus infection
   ➢ Testing is not mandatory to be eligible. Testing for HCV should be considered if the patient is at risk for having undiagnosed HCV (e.g. history of injection drug use).
10. Malignant disease, other than that being treated in this study. Exceptions to this exclusion include the following: malignancies that were treated curatively and have not recurred within 2 years prior to study treatment; completely resected basal cell and squamous cell skin cancers; any malignancy considered to be indolent and that has never required therapy; and completely resected carcinoma in situ of any type.
11. Systemic anti-cancer therapy within 2 weeks of the first dose of study treatment. For cytotoxic agents that have major delayed toxicity, e.g. mitomycin C and nitrosoureas, 4 weeks is indicated as washout period. For patients receiving anticancer immunotherapies such as CTLA-4 antagonists, 6 weeks is indicated as the washout period.
12. Active diarrhea CTCAE grade 2 or a medical condition associated with chronic diarrhea (such as irritable bowel syndrome, inflammatory bowel disease)
13. Presence of 2: CTCAE grade 2 toxicity (except alopecia, peripheral neuropathy and ototoxicity, which are excluded if >= CTCAE grade 3) due to prior cancer therapy.
14. Active infection requiring systemic antibiotic therapy.
15. Active ulceration of the upper GI tract or GI bleeding
16. Active bleeding diathesis or on oral anti-vitamin K medication (except low-dose warfarin and aspirin or equivalent, as long as the INR <= 2.0)
17. Active autoimmune disease, motor neuropathy considered of autoimmune origin, and other CNS autoimmune disease
18. Patients requiring concomitant immunosuppressive agents or chronic treatment with corticoids except:
   ➢ replacement dose steroids in the setting of adrenal insufficiency
   ➢ topical, inhaled, nasal and ophthalmic steroids are allowed
19. Use of any live vaccines against infectious diseases (e.g. influenza, varicella, pneumococcus) within 4 weeks of initiation of study treatment (NB the use of live vaccines is not allowed through the whole duration of the study)
20. Use of hematopoietic colony-stimulating growth factors (e.g. G-CSF, GMCSF, M-CSF) < 2 weeks prior start of study drug. An erythroid stimulating agent is allowed as long as it was initiated at least 2 weeks prior to the first dose of study treatment.
21. Major surgery within 2 weeks of the first dose of study treatment (NB mediastinoscopy, insertion of a central venous access device, or insertion of a feeding tube are not considered major surgery).
22. Radiotherapy within 2 weeks of the first dose of study drug, except for palliative radiotherapy to a limited field, such as for the treatment of bone pain or a focally painful tun1or mass. To allow for assessment of response to treatment, patients must have remaining measurable disease that has not been irradiated
23. Participation in an interventional, investigational study within 2 weeks of the first dose of study treatment.
24. Any medical condition that would, in the investigator's judgment, prevent the patient's participation in the clinical study due to safety concerns, compliance with clinical study procedures or interpretation of study results.
25. Sexually active males unless they use a condom during intercourse while taking drug and for 90 days after stopping study treatment and should not father a child in this period. A condom is required to be used also by vasectomized men in order to prevent delivery of the drug via seminal fluid.
26. Pregnant or lactating women, where pregnancy is defined as the state of a female after conception and until the termination of gestation, confirmed by a positive hCG laboratory test. In rare cases of an endocrine-secreting tumor, hCG levels may be above normal limits but with no pregnancy in the patient. In these cases, there should be a repeat serum hCG test (with a non-rising result) and a vaginal/pelvic ultrasound to rule out pregnancy. Upon confirmation of results and discussion with the Medical representative, these patients may enter the study.
27. Women of child-bearing potential, defined as all women physiologically capable of becoming pregnant, unless they are using highly effective methods of contraception during study treatment and for 90 days after the last any dose of study treatment. Highly effective contraception methods include:
   - Total abstinence (when this is in line with the preferred and usual lifestyle of the patient. Periodic abstinence (e.g., calendar, ovulation, symptothermal, post-ovulation methods) and withdrawal are not acceptable methods of contraception
   - Female sterilization (have had surgical bilateral oophorectomy with or without hysterectomy), total hysterectomy or tubal ligation at least 6 weeks before taking study treatment. In case of oophorectomy alone, only when the reproductive status of the woman has been confirmed by follow up hormone level assessment
   - Male sterilization (at least 6 months prior to screening). For female patients on the study the vasectomized male partner should be the sole partner for that patient.
   - Use of oral (estrogen and progesterone), injected or implanted combined hormonal methods of contraception or placement of an intrauterine device (IUD) or intrauterine system (IUS) or other forms of hormonal contraception that have comparable efficacy (failure rate <1%), for example hormone vaginal ring or transdermal hormone contraception.
      ➢ In case of use of oral contraception, women should have been stable on the same pill for a minimum of 3 months before taking study treatment.
      ➢ Women are considered post-menopausal and not of child bearing potential if they have had 12 months of natural (spontaneous) amenorrhea with an appropriate clinical profile (e.g. age appropriate, history of vasomotor symptoms) or have had surgical bilateral oophorectomy (with or without hysterectomy) or tubal ligation at least 6 weeks ago. In the case of oophorectomy alone, only when the reproductive status of the woman has been confirmed by follow up hormone level assessment is she considered not of child bearing potential.

### Dose-Limiting Toxicities and Dose modification guidelines

A dose-limiting toxicity (DLT) is defined as any of the following events thought to be at least possibly related to ADC per investigator judgment that occurs during the 21-day DLT evaluation period. Toxicity that is clearly and directly related to the primary disease or to another etiology is excluded from this definition.

### DLT Definitions

A **hematologic DLT** is defined as:
▪ Grade 3 or 4 febrile neutropenia or neutropenic infection
▪ Grade 4 neutropenia lasting >7 days
▪ Grade 4 thrombocytopenia
▪ Grade 3 thrombocytopenia with clinically significant bleeding, or Grade 3 thrombocytopenia requiring a platelet transfusion
▪ Grade 3 anemia that requires transfusion
▪ Grade 4 anemia

A **non-hematologic DLT** is defined as:
▪ Grade 4 non-hematologic toxicity
▪ Grade 3 non-hematologic toxicity lasting >3 days despite optimal supportive care or medical intervention
▪ A case of Hy's law (AST and/or ALT > 3x ULN and bilirubin > 2x ULN, and without initial findings of cholestasis (serum alkaline phosphatase (ALP) activity < 2x ULN) and no other reason that could explain the combination of increased transaminases and serum total bilirubin, such as viral hepatitis A, B, or C, preexisting or acute liver disease, or another drug capable of causing the observed injury)
▪ Grade 3 or higher hypersensitivity/infusion-related reaction (regardless of premedication). A grade 3 hypersensitivity / infusion-related reaction that resolves within 8 hours after onset with appropriate clinical management does not qualify as a DLT.
▪ LVEF decrease to < 40% or >20% decrease from baseline
▪ Grade 4 tumor lysis syndrome (Grade 3 TLS will not constitute DLT unless it leads to irreversible end-organ damage)

The following conditions are not considered non-hematologic DLT:
- Grade 3 fatigue for ≤ 7 days
- Grade 3 diarrhea, nausea, or vomiting in the absence of premedication that responds to therapy and improves by at least 1 grade within 3 days for Grade 3 events or to ≤ Grade 1 within 7 days.
- AST or ALT elevation ≥ 5 x ULN but ≤ 8 x ULN, without concurrent elevation in bilirubin, that downgrades to ≤ Grade 2 within 5 days after onset.
- Grade 3 serum lipase or serum amylase for ≤ 7 days if without clinical signs or symptoms of pancreatitis

Patients who experience a DLT that resolves or stabilizes with appropriate medical management may continue treatment at the *discretion* of the investigator in consultation with the sponsor.

### Dose modifications

Guidelines for management of specific toxicities are detailed in the table below. For management of events not specified in the tables, the following may serve as a guidance to investigators:

| **AE Grade** | **ADC Management Guideline** |
|---|---|
| **1** | No dose adjustment is required. |
| **2** | First occurrence: |
| | Consider holding one or both drugs until improvement to ≤ Grade 1 or baseline. Up to 1 dose of one or both drugs may be skipped to permit improvement. If improvement to ≤ Grade 1 or baseline occurs within 21 days from the last scheduled (but missed) dose of one or both drugs, continue one or both drugs at the original assigned dose level in subsequent treatment cycles. |
| | If improvement to ≤ Grade 1 or baseline does not occur within 21 days from the last scheduled (but missed) dose, permanently discontinue one or both drugs. |
| | Second occurrence: |
| | Hold one or both drugs until improvement to ≤ Grade 1 or baseline. Up to 1 dose of one or both drugs may be skipped to permit resolution. If improvement to ≤ Grade 1 or baseline occurs within 21 days from the last scheduled (but missed) dose, continue one or both drugs at 1 dose level below the original assigned dose in subsequent treatment cycles. If improvement to ≤ Grade 1 or baseline does not occur within 21 days from the last scheduled (but missed) dose, permanently discontinue one or both drugs. |
| | Third occurrence: |
| | Permanently discontinue one or both drugs. |
| **3** | First occurrence: |
| | Hold one or both drugs until improvement to ≤ Grade 1 or baseline. Up to 1 dose of one or both drugs may be skipped to permit improvement, then continue at 1 dose level below the original assigned dose in subsequent treatment cycles. |
| | Second occurrence: |
| | Permanently discontinue one or both drugs |
| **4** | Permanently discontinue one or both drugs. |

### Example 3

### Methods

MTT proliferation assay and IC50 calculation on cell lines exposed (96h) to increasing ADCx19 concentrations. Pearson correlation (r): calculated for IC50s vs cell surface CD19 expression levels (absolute fluorescence quantitation with Quantum Simply Cellular microspheres; non-absolute, data from PMID 29298756) and vs RNA levels (Illumina HT-12 arrays and HTG EdgeSeq Oncology Biomarker Panel data from PMID 29066507). Synergy at 96h was assessed by Chou-Talalay combination index (CI) (synergism CI<0.9, additive CI=0.9-1.1, antagonism/no benefit CI> 1.1) on 2 activated B cell like (ABC) DLBCL (OCI-LY-3, TMD8) and 2 germinal center (GCB) DLBCL (VAL, WSU-DLCL2).

### Results

Median ADCx19 IC50 was 4 pM (95%C.I, 2-10 pM) in 48 B-cell lymphoma lines, and, as expected based on CD19 expression pattern, over 800 times higher in 9 T-cell lymphoma lines (3.5 nM; 95%C.I, 0.8-11 nM). Focusing on B-cell lymphomas, ADCx19 in vitro activity was correlated with its target expression measured both at cell surface protein level [(absolute quantitation, n=40, r -0.37 P 0.02; non-absolute quantitation, n=42, -0.48, P 0.001] and RNA level [(arrays, n=39, -0.69 P <0.001; HTG, n=31, -0.73 P 0.001]. In DLBCL, the presence of BCL2 and MYC translocations or TP53 inactivation did not affect the sensitivity to ADCx19.

ADCx19was then combined in GCB- and ABC- DLBCL cell lines with the BCL2 inhibitor venetoclax

Synergism in all cell lines was achieved combining ADCx19 with venetoclax

### Cell line: OCI-LY3

### RRID cell accession identifier: CVCL_8800

| **ADCx19 (pM)** | **Venetoclax (nM)** | **Fa** | **Cl** | **ADCx19 (pM)** | **Venetoclax (nM)** | **Fa** | **Cl** |
|---|---|---|---|---|---|---|---|
| 0.02 | 2.44 | 0.88 | 0.55 | 1.56 | 156.25 | 0.37 | 0.51 |
| 0.02 | 9.77 | 0.75 | 0.46 | 1.56 | 625 | 0.24 | 0.64 |
| 0.02 | 39.06 | 0.63 | 0.74 | 1.56 | 2500 | 0.15 | 0.96 |
| 0.02 | 156.25 | 0.39 | 0.57 | 6.25 | 2.44 | 0.75 | 0.71 |
| 0.02 | 625 | 0.25 | 0.75 | 6.25 | 9.77 | 0.57 | 0.27 |
| 0.02 | 2500 | 0.17 | 1.36 | 6.25 | 39.06 | 0.48 | 0.34 |
| 0.1 | 2.44 | 0.83 | 0.29 | 6.25 | 156.25 | 0.30 | 0.29 |
| 0.1 | 9.77 | 0.73 | 0.40 | 6.25 | 625 | 0.19 | 0.40 |
| 0.1 | 39.06 | 0.61 | 0.64 | 6.25 | 2500 | 0.13 | 0.72 |
| 0.1 | 156.25 | 0.38 | 0.51 | 25 | 2.44 | 0.44 | 0.26 |
| 0.1 | 625 | 0.25 | 0.71 | 25 | 9.77 | 0.36 | 0.17 |
| 0.1 | 2500 | 0.17 | 1.23 | 25 | 39.06 | 0.23 | 0.09 |
| 0.39 | 2.44 | 0.92 | 1.67 | 25 | 156.25 | 0.16 | 0.09 |
| 0.39 | 9.77 | 0.76 | 0.56 | 25 | 625 | 0.10 | 0.13 |
| 0.39 | 39.06 | 0.59 | 0.54 | 25 | 2500 | 0.07 | 0.24 |
| 0.39 | 156.25 | 0.40 | 0.60 | 100 | 2.44 | 0.19 | 0.14 |
| 0.39 | 625 | 0.24 | 0.69 | 100 | 9.77 | 0.16 | 0.09 |
| 0.39 | 2500 | 0.15 | 1.01 | 100 | 39.06 | 0.12 | 0.06 |
| 1.56 | 2.44 | 0.86 | 0.85 | 100 | 156.25 | 0.08 | 0.04 |
| 1.56 | 9.77 | 0.71 | 0.42 | 100 | 625 | 0.04 | 0.03 |
| 1.56 | 39.06 | 0.53 | 0.38 | 100 | 2500 | 0.03 | 0.06 |

### Cell line: TMD8

### RRID cell accession identifier: CVCL_A442

### Reference: Tohda et al., Leuk. Res. 30:1385-1390(2006)

| **ADCx19 (pM)** | **Venetoclax (nM)** | **Fa** | **Cl** | **ADCx19 (pM)** | **Venetoclax (nM)** | **Fa** | **Cl** |
|---|---|---|---|---|---|---|---|
| 0.02 | 2.44 | 0.98 | 3.70 | 0.39 | 156.25 | 0.81 | 1.63 |
| 0.02 | 9.77 | 0.96 | 3.64 | 0.39 | 625 | 0.68 | 1.40 |
| 0.02 | 39.06 | 0.87 | 1.02 | 0.39 | 2500 | 0.55 | 1.83 |
| 0.02 | 156.25 | 0.77 | 0.93 | 1.56 | 2.44 | 0.79 | 0.30 |
| 0.02 | 625 | 0.72 | 2.09 | 1.56 | 9.77 | 0.77 | 0.33 |
| 0.02 | 2500 | 0.59 | 2.45 | 1.56 | 39.06 | 0.68 | 0.33 |
| 0.1 | 2.44 | 0.94 | 0.47 | 1.56 | 156.25 | 0.64 | 0.48 |
| 0.1 | 9.77 | 0.87 | 0.29 | 1.56 | 625 | 0.51 | 0.50 |
| 0.1 | 39.06 | 0.88 | 1.19 | 1.56 | 2500 | 0.40 | 0.63 |
| 0.1 | 156.25 | 0.77 | 0.93 | 6.25 | 2.44 | 0.31 | 0.63 |
| 0.1 | 625 | 0.70 | 1.75 | 6.25 | 9.77 | 0.28 | 0.61 |
| 0.1 | 2500 | 0.59 | 2.46 | 6.25 | 39.06 | 0.21 | 0.55 |
| 0.39 | 2.44 | 0.95 | 0.82 | 6.25 | 156.25 | 0.19 | 0.54 |
| 0.39 | 9.77 | 0.87 | 0.32 | 6.25 | 625 | 0.10 | 0.44 |
| 0.39 | 39.06 | 0.81 | 0.47 | 6.25 | 2500 | 0.08 | 0.42 |

### Cell line: VAL

### RRID cell accession identifier: CVCL_1819

| **ADCx19 (pM)** | **Venetoclax (nM)** | **Fa** | **Cl** | **ADCx19 (pM)** | **Venetoclax (nM)** | **Fa** | **Cl** |
|---|---|---|---|---|---|---|---|
| 0.02 | 2.44 | 0.73 | 0.70 | 0.39 | 2.44 | 0.52 | 0.77 |
| 0.02 | 9.77 | 0.46 | 0.81 | 0.39 | 9.77 | 0.32 | 0.72 |
| 0.02 | 39.06 | 0.21 | 0.98 | 0.39 | 39.06 | 0.13 | 0.66 |
| 0.1 | 2.44 | 0.66 | 0.64 | 1.56 | 2.44 | 0.28 | 1.04 |
| 0.1 | 9.77 | 0.42 | 0.77 | 1.56 | 9.77 | 0.16 | 0.74 |
| 0.1 | 39.06 | 0.19 | 0.92 | 1.56 | 39.06 | 0.05 | 0.43 |

### Cell line: WSU-DLCL2

### RRID cell accession identifier: CVCL_1902

| **ADCx19 (pM)** | **Venetoclax (nM)** | **Fa** | **Cl** | **ADCx19 (pM)** | **Venetoclax (nM)** | **Fa** | **Cl** |
|---|---|---|---|---|---|---|---|
| 0.02 | 2.44 | 0.87 | 0.21 | 1.56 | 625 | 0.65 | 1.76 |
| 0.02 | 9.77 | 0.91 | 1.98 | 1.56 | 2500 | 0.54 | 1.77 |
| 0.02 | 39.06 | 0.89 | 5.03 | 1.56 | 10000 | 0.14 | 0.03 |
| 0.02 | 156.25 | 0.85 | 7.56 | 6.25 | 2.44 | 0.61 | 0.70 |
| 0.02 | 625 | 0.77 | 6.89 | 6.25 | 9.77 | 0.57 | 0.52 |
| 0.02 | 2500 | 0.68 | 8.49 | 6.25 | 39.06 | 0.46 | 0.22 |
| 0.02 | 10000 | 0.13 | 0.03 | 6.25 | 156.25 | 0.43 | 0.19 |
| 0.1 | 2.44 | 0.87 | 0.40 | 6.25 | 625 | 0.38 | 0.18 |
| 0.1 | 9.77 | 0.89 | 1.58 | 6.25 | 2500 | 0.32 | 0.20 |
| 0.1 | 39.06 | 0.87 | 2.72 | 6.25 | 10000 | 0.13 | 0.03 |
| 0.1 | 156.25 | 0.84 | 6.53 | 25 | 2.44 | 0.32 | 0.23 |
| 0.1 | 625 | 0.79 | 10.42 | 25 | 9.77 | 0.27 | 0.15 |
| 0.1 | 2500 | 0.65 | 6.18 | 25 | 39.06 | 0.24 | 0.10 |
| 0.1 | 10000 | 0.13 | 0.03 | 25 | 156.25 | 0.22 | 0.08 |
| 0.39 | 2.44 | 0.88 | 1.21 | 25 | 625 | 0.19 | 0.07 |
| 0.39 | 9.77 | 0.89 | 2.50 | 25 | 2500 | 0.18 | 0.07 |
| 0.39 | 39.06 | 0.85 | 2.54 | 25 | 10000 | 0.07 | 0.01 |
| 0.39 | 156.25 | 0.80 | 2.98 | 100 | 2.44 | 0.23 | 0.35 |
| 0.39 | 625 | 0.73 | 4.18 | 100 | 9.77 | 0.20 | 0.26 |
| 0.39 | 2500 | 0.63 | 4.85 | 100 | 39.06 | 0.17 | 0.17 |
| 0.39 | 10000 | 0.13 | 0.03 | 100 | 156.25 | 0.15 | 0.13 |
| 1.56 | 2.44 | 0.85 | 2.47 | 100 | 625 | 0.14 | 0.11 |
| 1.56 | 9.77 | 0.82 | 1.87 | 100 | 2500 | 0.13 | 0.10 |
| 1.56 | 39.06 | 0.81 | 2.34 | 100 | 10000 | 0.04 | 0.01 |
| 1.56 | 156.25 | 0.77 | 2.71 | | | | |

### Conclusion

The strong single agent in vitro anti-lymphoma activity of ADCx19 correlated with its target expression and supports the currently on-going clinical studies in relapsed/refractory DLBCL. The novel venetoclax combination data indicates clinical synergy.

## Claims

1. ADCx19 for use in a method for treating a cancer in an individual, the method comprising administering to the individual an effective amount of ADCx19 and venetoclax;
wherein ADCx19 has the chemical structure: 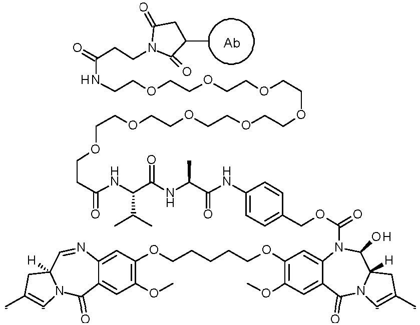
wherein Ab represents an antibody comprising a VH domain having the sequence of SEQ ID NO. 2, and a VL domain having the sequence of SEQ ID NO. 8.

2. The ADCx19 for use according to claim 6, wherein the method comprises administering ADCx19 before venetoclax, simultaneous with venetoclax, or after venetoclax.

3. The ADCx19 for use according to either one of claims 1 and 2, wherein the method further comprises administering a chemotherapeutic agent.

4. The ADCx19 for use according to any one of claims 1 to 3, wherein the individual is human.

5. The ADCx19 for use according to any one of claims 1 to 4, wherein the individual has or has been determined to have a cancer which expresses CD19.

6. The ADCx19 for use according to any previous claim, wherein the individual is being treated, or has been treated, with venetoclax.

7. The ADCx19 for use according to any previous claim, wherein the individual is refractory to treatment, or further treatment, with venetoclax.

8. The ADCx19 for use of any one of claims 1 to 7, wherein the cancer is selected from the group comprising: non-Hodgkin's Lymphoma, including diffuse large B-cell lymphoma (DLBCL), follicular lymphoma, (FL), Mantle Cell lymphoma (MCL), chronic lymphatic lymphoma (CLL), and Marginal Zone B-cell lymphoma (MZBL), and leukemias such as Hairy cell leukemia (HCL), Hairy cell leukemia variant (HCL-v), and Acute Lymphoblastic Leukaemia (ALL) such as Philadelphia chromosome-positive ALL (Ph+ALL) or Philadelphia chromosome-negative ALL (Ph-ALL).

9. The ADCx19 for use according to any one of claim 1 to 8, wherein the individual is selected for treatment if the individual is or has been treated with venetoclax, optionally wherein the individual is selected for treatment if they are refractory to treatment, or further treatment, with venetoclax.

## Patentansprüche

1. ADCx19 zur Verwendung in einem Verfahren zur Behandlung einer Krebserkrankung in einem Individuum, wobei das Verfahren das Verabreichen einer wirksamen Menge von ADCx19 und Venetoclax an das Individuum umfasst;
wobei das ADCx19 die folgende chemische Struktur aufweist: 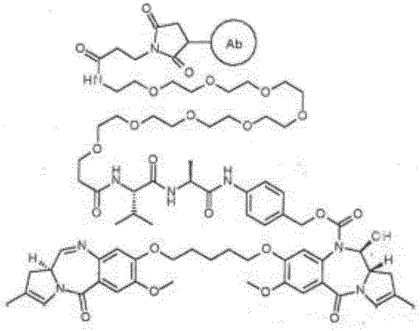 worin Ab für einen Antikörper steht, der eine VH-Domäne, welche die Sequenz von SEQ ID NO: 2 aufweist, und eine VL-Domäne, welche die Sequenz von SEQ ID NO: 8 aufweist, umfasst.

2. ADCx19 zur Verwendung nach Anspruch 1, wobei das Verfahren das Verabreichen von ADCx19 vor Venetoclax, gleichzeitig mit Venetoclax oder nach Venetoclax umfasst.

3. ADCx19 zur Verwendung nach einem der beiden Ansprüche 1 und 2, wobei das Verfahren weiters das Verabreichen eines Chemotherapeutikums umfasst.

4. ADCx19 zur Verwendung nach einem der Ansprüche 1 bis 3, wobei das Individuum ein Mensch ist.

5. ADCx19 zur Verwendung nach einem der Ansprüche 1 bis 4, wobei bei dem Individuum bestimmt wurde, dass es an einer Krebserkrankung leidet, die CD19 exprimiert.

6. ADCx19 zur Verwendung nach einem der vorangegangenen Ansprüche, wobei das Individuum mit Venetoclax behandelt wird oder damit behandelt wurde.

7. ADCx19 zur Verwendung nach einem der vorangegangenen Ansprüche, wobei das Individuum gegenüber der Behandlung oder einer weiteren Behandlung mit Venetoclax refraktär ist.

8. ADCx19 zur Verwendung nach einem der Ansprüche 1 bis 7, wobei die Krebserkrankung aus der aus den folgenden bestehenden Gruppe ausgewählt ist: Non-Hodgkin-Lymphom, einschließlich von diffusem großem B-Zell-Lymphom (DLBCL), follikulärem Lymphom (FL), Mantelzellenlymphom (MCL), chronischem lymphatischem Lymphom (CLL) und Marginalzonen-B-Zell-Lymphom (MZBL), und Leukämien, wie z. B. Haarzellenleukämie (HCL), Haarzellenleukämie-Variante (HCL-v) und akute lymphoblastische Leukämie (ALL), wie z. B. Philadelphia-Chromosom-positive ALL (Ph+ALL) oder Philadelphia-Chromosomnegative ALL (Ph-ALL).

9. ADCx19 zur Verwendung nach einem der Ansprüche 1 bis 8, wobei das Individuum zur Behandlung ausgewählt wird, wenn das Individuum mit Venetoclax behandelt wird oder damit behandelt wurde, wobei das Individuum gegebenenfalls zur Behandlung ausgewählt wird, wenn es gegenüber einer Behandlung oder einer weiteren Behandlung mit Venetoclax refraktär ist.

## Revendications

1. ADCx19 pour utilisation dans un procédé de traitement d'un cancer chez un individu, le procédé comprenant l'administration à l'individu d'une quantité efficace d'ADCx19 et de vénétoclax ;
dans lequel l'ADCx19 présente la structure chimique : 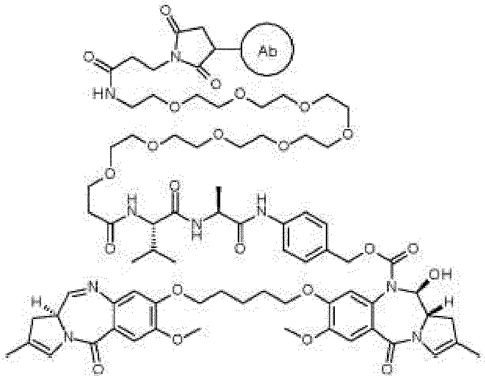
dans lequel Ab représente un anticorps comprenant un domaine VH présentant la séquence de SEQ ID NO. 2, et un domaine VL présentant la séquence de SEQ ID NO. 8.

2. ADCx19 pour utilisation selon la revendication 6, dans lequel le procédé comprend l'administration de l'ADCx19 avant le vénétoclax, en même temps que le vénétoclax ou après le vénétoclax.

3. ADCx19 pour utilisation selon l'une quelconque des revendications 1 et 2, dans lequel le procédé comprend en outre l'administration d'un agent chimiothérapeutique.

4. ADCx19 pour utilisation selon l'une quelconque des revendications 1 à 3, dans lequel l'individu est un être humain.

5. ADCx19 pour utilisation selon l'une quelconque des revendications 1 à 4, dans lequel l'individu présente ou a été déterminé comme présentant un cancer qui exprime CD19.

6. ADCx19 pour utilisation selon l'une quelconque des revendications précédentes, dans lequel l'individu est traité, ou a été traité, avec du vénétoclax.

7. ADCx19 pour utilisation selon l'une quelconque des revendications précédentes, dans lequel l'individu est réfractaire au traitement, ou à un traitement supplémentaire, par vénétoclax.

8. ADCx19 pour utilisation selon l'une quelconque des revendications 1 à 7, dans lequel le cancer est choisi dans le groupe comprenant : un lymphome non hodgkinien, y compris un lymphome diffus à grandes cellules B (DLBCL), un lymphome folliculaire (FL), un lymphome à cellules du manteau (MCL), un lymphome lymphatique chronique (CLL) et un lymphome à cellules B de zone marginale (MZBL), et des leucémies telles que la leucémie à tricholeucocytes (HCL), une variante de la leucémie à tricholeucocytes (HCL-v) et une leucémie lymphoblastique aiguë (LLA) telle que la LLA à chromosome Philadelphie positif (Ph+ALL) ou la LLA à chromosome Philadelphie négatif (Ph-ALL).

9. ADCx19 pour utilisation selon l'une quelconque des revendications 1 à 8, dans lequel l'individu est sélectionné pour un traitement si l'individu est ou a été traité avec du vénétoclax, facultativement dans lequel l'individu est sélectionné pour un traitement s'il est réfractaire au traitement, ou à un traitement supplémentaire, avec du vénétoclax.
